(19) Europäisches Patentamt
European Patent Office
Office européen des brevets

(11) Numéro de publication: **0 008 965**
**B1**

(12) **FASCICULE DE BREVET EUROPEEN**

(45) Date de publication du fascicule du brevet: **05.05.82**

(51) Int. Cl.³: **C 07 H 15/22,**
A 61 K 31/70

(21) Numéro de dépôt: **79400541.3**

(22) Date de dépôt: **31.07.79**

# (54) Nouveaux aminoglycosides dérivés de la désoxystreptamine et leurs sels, leur procédé de préparation, leur application à titre de médicaments et les compositions pharmaceutiques les renfermant.

(30) Priorité: **06.09.78 FR 7825604**

(43) Date de publication de la demande:
**19.03.80 Bulletin 80/6**

(45) Mention de la délivrance du brevet:
**05.05.82 Bulletin 82/18**

(84) Etats contractants désignés:
**AT BE CH DE GB NL SE**

(56) Documents cités:
**FR - A - 2 318 648**
**FR - A - 2 358 156**

(73) Titulaire: **ROUSSEL-UCLAF**
**102, route de Noisy Boîte postale no.9**
**F-93230 Romainville (FR)**

(72) Inventeur: **Gasc, Jean-Claude**
**6, place Georges Lyssandre**
**F-93140 Bondy (FR)**
Inventeur: **Rettien, Claude**
**28, rue de la Fédération**
**F-93100 Montreuil sous Bois (FR)**

(74) Mandataire: **Niel, Michel et al,**
**boîte postale no 9 102, route de Noisy**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 008 965 B1

Nouveaux aminoglycosides dérivés de la désoxystreptamine et leurs sels, leur procédé de préparation, leur application à titre de médicaments et les compositions pharmaceutiques les renfermant.

La présente invention concerne de nouveaux aminoglycosides dérivés de la désoxystreptamine et leurs sels, leur procédé de préparation, leur application à titre médicaments et les compositions pharmaceutiques les renfermant.

On connait déjà (BF 2318 648) un dérivé de la famille des aminoglycosides à savoir la $N_1$/L(—)2-hydroxy 4-amino butyroyl/″-0/2′,6′-diamino 2′,6′-didésoxy $\alpha$,D glucopyranosyle/6-0/3″-méthylamino 3″,4″,6″-tridésoxy $\alpha$,D-xylohexopyranosyl/2-désoxy streptamine, produit qui comporte des radicaux hydroxyle en positions 3′ et 4′.

De même le brevet français BF 2 358156 décrit un analogue de la désoxy streptamine la 4-0/2′,6′-diamino 2′,3′,6′-tridésoxy $\alpha$,D-ribohexopyranosyl/6-0-/3″-N-méthylamino 3″-désoxy $\alpha$,D-glucopyranosyl/2-desoxy streptamine, produit qui ne comporte pas de chaine 4-hydroxy 4-amino butyroyl.

L'invention a pour objet de nouveaux aminoglycosides dérivés de la désoxystreptamine, ainsi que leurs sels d'addition avec les acides minéraux ou organiques, caractérisés en ce qu'ils répondent à la formule générale I:

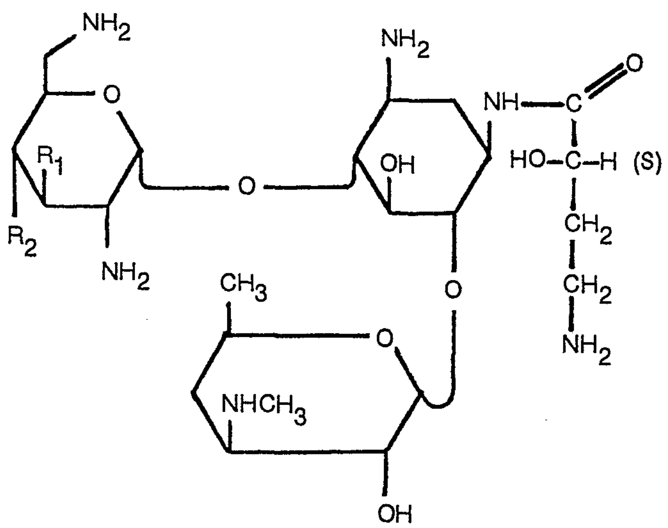

I

dans laquelle les substituants $R_1$ et $R_2$ représentent un atome d'hydrogène ou un groupement hydroxyle, l'un au moins de ces substituants n'étant pas un groupement hydroxyle.

Le symbole (S) est l'expression d'une configuration stérique des composés organiques/R. S. Cahn, C. K. Ingold et V. Prelog's "Experientia" Vol. 12 pages 81—94 (1956) /.

Les sels peuvent être, par exemple, un chlorhydrate, un bromhydrate, un nitrate, un sulfate, un phosphate, un acétate, un formiate, un benzoate, un maléate, un fumarate, un succinate, un tartrate, un citrate, un oxalate, un benzylate, un glyoxylate, un aspartate, un alcane sulfonate ou un arylsulfonate.

L'invention a plus particulièrement pour objet:

— 1′O-/2,6-diamino 2,3,6-tridésoxy $\alpha$,D-ribohexopyranosyl (1→4)/-O-/3-méthylamino 3,4,6-tridésoxy $\alpha$,D-xylohexopyranosyl (1→6)/$N_1$ (4-amino 2(S) hydroxy 1-oxo) butyl 2-désoxy D-streptamine et son sulfate,

— 1′O-/2,6-diamino 2,4,6-tridésoxy $\alpha$,D-xylohexopyranosyl (1→4)/-O-/3-méthylamino 3,4,6-tridésoxy $\alpha$,D-xylohexopyranosyl (1→6)/$N_1$ (4-amino 2(S)-hydroxy 1-oxo) butyl 2-désoxy D-streptamine et son sulfate,

— 1′ O-/2,6-diamino 2,3,4,6-tétradésoxy $\alpha$,D-érythrohexopyranoysl (1→4)/-O-/3-méthylamino 3,4,6-tridésoxy $\alpha$,D-xylohexopyranosyl (1→6)/$N_1$1 (4-amino 2(S)-hydroxy 1-oxo)butyl 2-désoxy D-streptamine et son sulfate.

L'invention a également pour objet un procédé de préparation des produits de formule I ci-dessus et de leurs sels.

Ce procédé est caractérisé en ce que l'on fait réagir un produit de formule II:

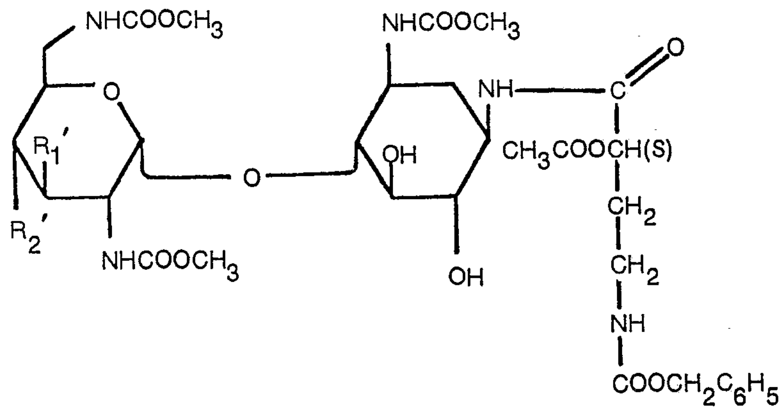

II

dans laquelle les substituants $R'_1$ et $R'_2$ représentent un atome d'hydrogène ou un groupement benzoxy, l'un au moins de ces substituants n'étant pas un groupement benzoxy, avec le produit de formule III:

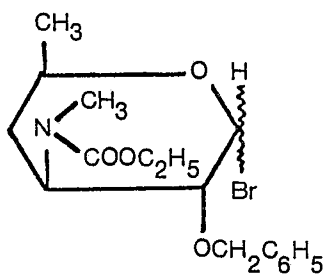

III

en présence d'un agent alcalin, pour obtenir un mélange d'anomères $\alpha$ et $\beta$ de formules IV et IV':

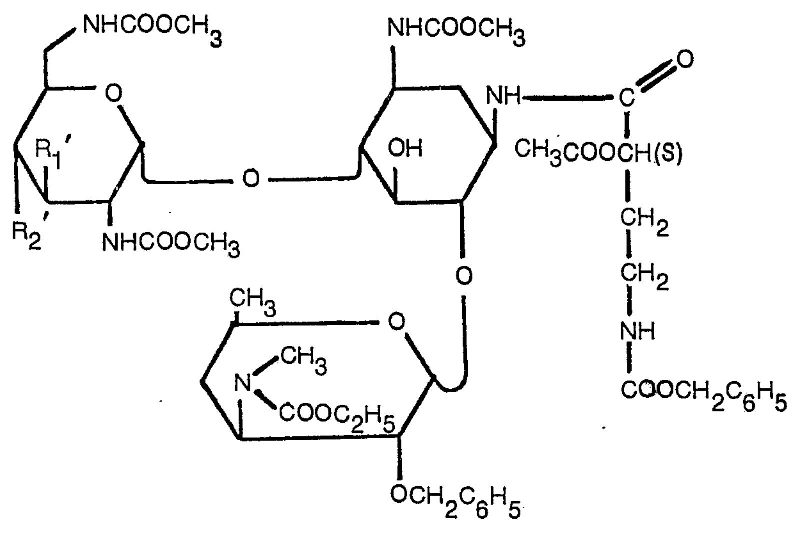

IV

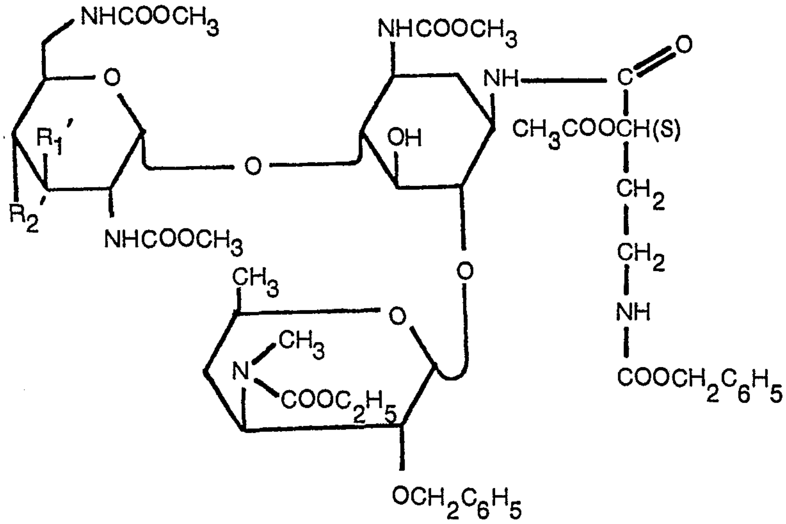

IV'

mélange dont on isole le produit de formule IV, produit de formule IV que l'on hydrolyse en milieu alcalin, dans un solvant organique, pour obtenir un produit de formule V:

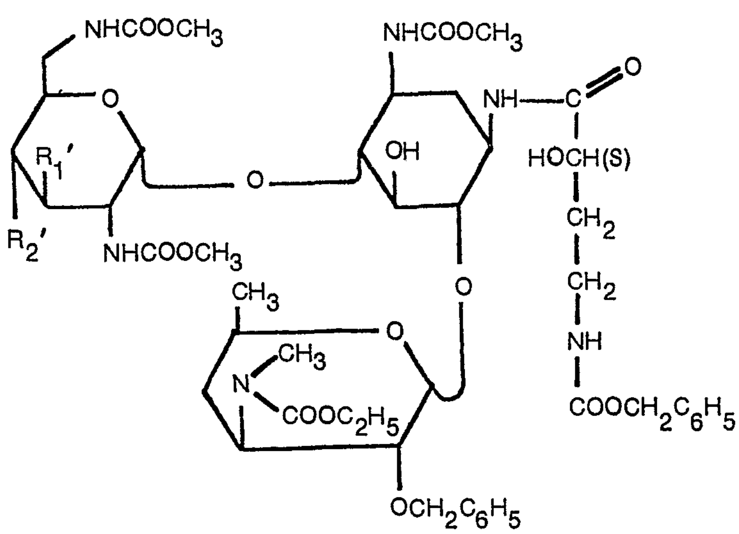

V

produit de formule V que l'on traite par l'hydrogène, en présence d'un catalyseur pour obtenir un produit de formule VI:

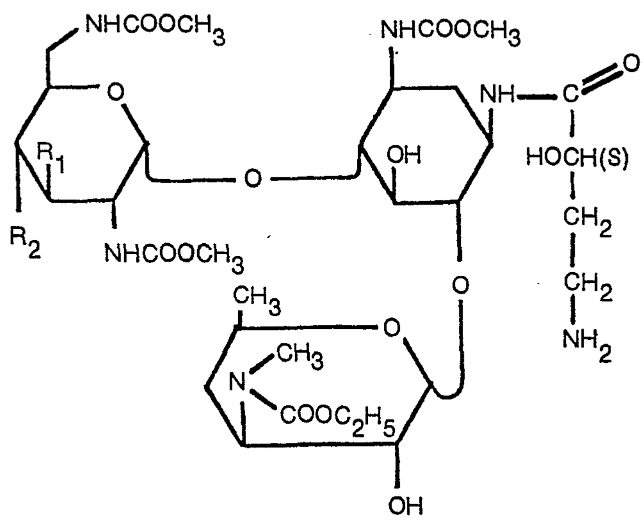

VI

dans laquelle les substituants $R_1$ et $R_2$ ont la signification déjà indiquée, produit de formule VI que l'on hydrolyse en milieu alcalin pour obtenir le produit de formule I cherché, produit de formule I que l'on salifie, si désiré, par action d'un acide minéral ou organique pour en formel le sel cherchée.

Dans un mode préférentiel d'exécution du procédé ci-dessus, on opère comme indiqué ci-après.

L'agent alcalin utilisé pour la réaction du produit de formule II avec le produit de formule III est de préférence la diisopropyléthylamine, mais on peut utiliser une autre base tertiaire comme la triéthylamine, en présence d'un ammonium quaternaire de tétraalkyle inférieur, comme le tétraéthyl ammonium.

La réaction s'opère dans un solvant organique qui est avantageusement le chlorure de méthylène, mais il est possible d'utiliser le dichloroéthane ou le diméthylformamide.

La séparation des produits de formules IV et IV' peut être réalisée par les méthodes habituelles; on utilise avantageusement la chromatographie sur silice, mais on peut également utiliser la chromatographie sur alumine, sur cellulose, sur silicate de magnésium ou encore la cristallisation fractionnée ou la méthode dite de séparation à contre-courant. On utilise pour mener à bien de telles séparations différents solvants ou mélanges de solvants organiques purs ou aqueux.

L'agent alcalin utilisé pour l'hydrolyse du produit de formule IV est, de préférence, l'hydroxyde de sodium, mais il est aussi possible d'utiliser les hydroxydes de potassium, de lithium ou de baryum. Le solvant organique est avantageusement composé d'un mélange d'un alcool tel l'éthanol ou le méthanol et de chlorure de méthylène ou de diméthylformamide.

L'agent d'hydrogénolyse que l'on fait agir sur le produit de formule V est de préférence l'hydrogène en présence de palladium comme catalyseur. On peut cependant utiliser d'autres catalyseurs, comme par exemple le nickel de Raney, les sels de palladium ou des dérivés du platine. Le solvant utilisé pour mener à bien la réaction est de préférence un solvant aqueux acide tel un mélange d'acide acétique ou propionique et d'eau.

L'agent alcalin utilisé pour hydrolyser le produit de formule VI est, dans les conditions préférentielles d'exécution de la réaction, l'hydroxyde de potassium, mais il est également possible d'utiliser l'hydroxyde de sodium.

La salification des produits de formule I peut être réalisée selon les méthodes usuelles par neutralisation totale ou partielle des cinq fonctions aminées des produits de formule I.

Elle peut être obtenue par action sur ces produits d'acides tels que, par exemple, les acides chlorhydrique, bromhydrique, nitrique, sulfurique, phosphorique, acétique, formique, benzoique, maléique, fumarique, succinique, tartrique, citrique, oxalique, benzylique, glyoxylique, aspartique, alcane sulfoniques et arylsulfoniques. Cette salification est réalisée, de préférence, dans un solvant ou un mélange de solvants tels que l'eau, l'éther éthylique, le méthanol ou l'acétone.

Le trait ondulé qui relie les substituants de l'atome de carbone en position 1 du cycle des produits de formule III indique que ces substituants peuvent se trouver en position $\alpha$ ou $\beta$ de ce cycle. Ce produit existe donc sous forme d'anomère $\alpha$ ou $\beta$, ou d'un mélange de ces anomères.

Les produits de formule I et leurs sels d'additon avec les acides possèdent une très intéressante activité antibiotique, d'une part sur les bactéries gram +, telles que les staphylocoques, les streptocoques et notamment les staphylocoques péniclline-résistants et, d'autre part, sur les bactéries gram — et notamment sur les bactéries coliformes.

Ces propriétés rendent aptes les produits de formule I ainsi que leurs sels thérapeutiquement compatibles, à être utilisés, à titre de médicaments, notamment dans le traitement des staphylococcies, telles que septicémies à staphylocoques, staphylococcies malignes de la face, staphylococcies cutanées, pyrodermites, plaies septiques et suppurantes, anthrax, phlegmons, érésypèles, staphylococcies aigués primitives ou postgrippales, bronchopneumonies, suppurations pulmonaires et collibacilloses.

Les produits de formule I peuvent également être utilisés dans le traitement des infections à Klebsiella, à Pseudomonas et à Entérobacter.

La présente invention a donc également pour objet, à titre de médicaments et notamment de médicaments antibiotiques, les produits de formule I et leurs sels thérapeutiquement compatibles.

La présente invention a plus spécialement pour objet, à titre de médicaments et notamment de médicaments antibiotiques, les produits des exemples ci-après.

L'invention s'étend aux compositions pharmaceutiques renfermant comme principe actif au moins un des médicaments définis ci-dessus.

Ces compositions pharmaceutiques peuvent être administrées par voie buccale, rectale, parentérale ou par voie locale en application topique sur la peau et les muqueuses.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, le gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 100 mg et 1 g par jour chez l'homme par voie parentérale pour le produit de l'exemple 2.

L'invention permet d'obtenir, à titre de produits industriels nouveaux, les produits de formule:

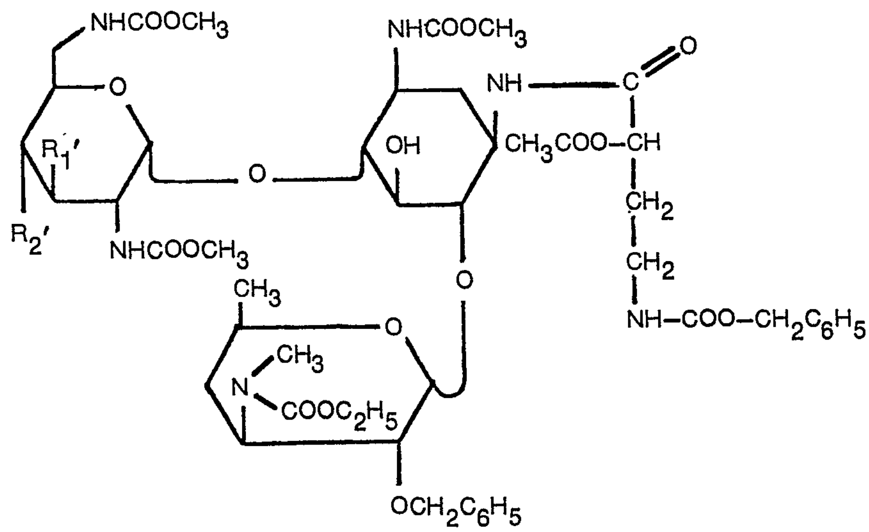

IV

dans laquelle les substituants $R'_1$ et $R'_2$ ont la signification déjà éconcée, les produits de formule:

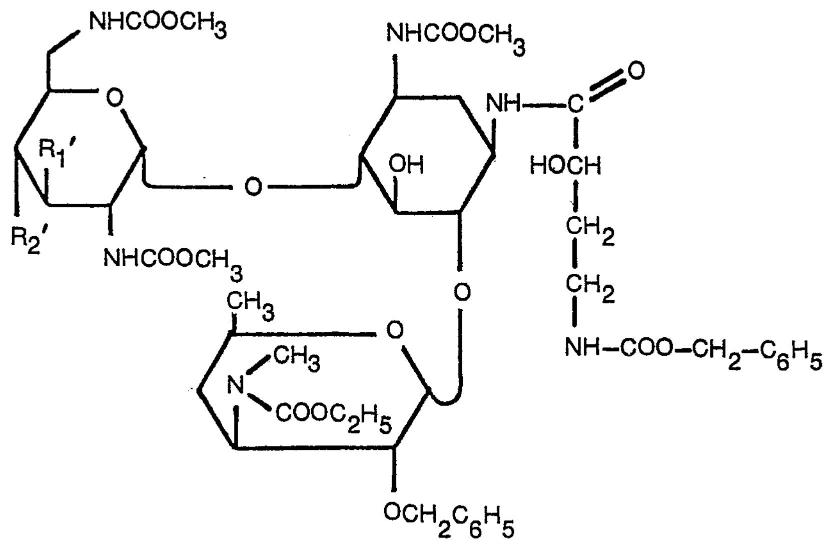

V

dans laquelle les substituants $R'_1$ et $R'_2$ ont la signification déjà énoncée, et les produits de formule:

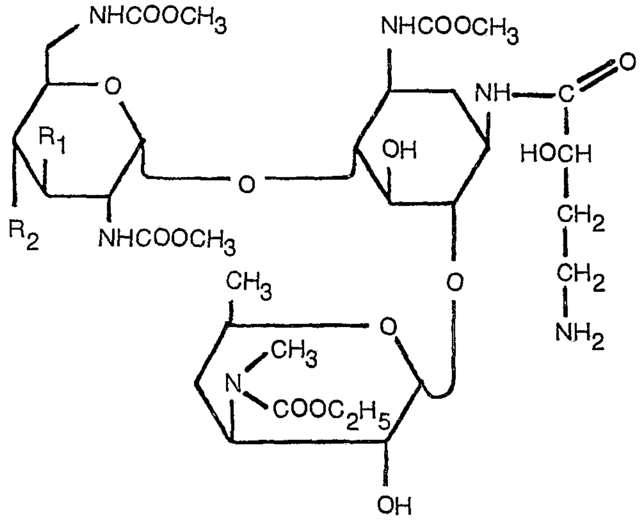

VI

dans laquelle les substituants $R'_1$ et $R'_2$ ont la signification déjà énoncée.

Les produits de formule II utilisés au départ peuvent être préparés selon un procédé analogue à celui décrit dans la demande de brevet français n° 2.202.078. Des exemples de préparation des produits de formule II sont décrits plus loin dans les exemples.

Les produits de formule III peuvent être préparés en traitant par l'acide bromhydrique le 1-O-acétyl, 2-O-benzyl 3-(N-carbéthoxy N-méthyl)amino 3,4,6-tridésoxy D-xylohexopyranose, décrit dans la demande de brevet français n° 2.290.908.

Les exemples suivants illustrent l'invention sans toutesfois lui conférer aucun caractère limitatif.

Exemple 1

1'-O-/2,6-diamino 2,3,6-tridesoxy $\alpha$,D-ribohexopyranosyl (1→4)/-O-/3-méthylamino 3,4,6-tridésoxy $\alpha$,D-xylohexopyranosyl (1→6)/N[1] (4-amino 2(S)-hydroxy 1-oxo) butyl 2-desoxy D streptamine.

*Stade A*

L'O-/3-(éthoxycarbonyl méthylamino)-2-O-phényl méthyl 3,4,6-tridésoxy $\alpha$,D-xylohexopyranosyl (1→6)/-O-/2,3,6-tridésoxy 2,6-bis/(méthoxycarbonyl)amino/4-O-phényl méthyl $\alpha$,D-ribohexopyranosyl (1→4)]N[1]-/2(S)-acétyloxy 1-oxo 4-/{(phényl méthoxy)carbonyl}amino/butyl/2-désoxy N[3]-(méthoxy carbonyl) D-streptamine.

On dissout dans 110 ml de chlorure de méthylène anhydre saturé d'acide bromhydrique, 2,4 g de 1-O-acétyl-2-O-benzyl 3-(N-carbéthoxy N-méthyl) amino 3,4,6-tridésoxy D-xylohexopyranose, agite pendant 45 minutes, évapore à sec; le résidu est repris par 90 ml de chlorure de méthylène et on y ajoute 1,39g de bromure de tétraéthylammonium, 1,19 g de diisopropyléthylamine et 3,73 g de 2-désoxy 4-O-/2,3,6-tridésoxy 2,6 bis/(méthoxycarbonyl)-amino/4-O-phényl méthyl $\alpha$,D-ribohexopyranosyl/N[1]-/2(S)-acétyloxy 1-oxo 4/{1(phényl méthoxy)carbonyl}amino/butyl/-N[3]-méthoxy-carbonyl D-streptamine, chauffe au réflux sous atmosphère inerte pendant 4 heures puis ajoute 0,5 équivalent du dérivé bromé comme préparé ci-dessus, 0,5 équivalent de bromure de tétraéthylammonium et 0,5 équivalent de diisopropyléthylamine et effectue le même rajout au bout de 22 heures, puis de 30 heures; après 46 heures de reflux on laisse refroidir, verse dans l'eau et extrait au chlorure de méthylène; les extraits sont sèchés et évaporés à sec, on obtient 8,256 g du produit brut cherché qui, purifié par chromatographie sur silice (éluant:chloroform-acétone 70/300) donne 3,5 g d'anomère $\alpha$, désiré. Rf = 0,30. L'anomère $\beta$ est caractérisé par un Rf = 0,20 dans les mêmes conditions.

*Stade B*

1'O-/3-(éthoxycarbonyl méthylamino)2-O-phényl méthyl 3,4,6-tridésoxy $\alpha$,D-xylohexopyranosyl (1→6)/-O-/2,3,6-tridésoxy 2,6-bis/(méthoxycarbonyl)amino/-4-O-phényl méthyl $\alpha$,D-ribohexopyranosyl (1→4)N[1]-/2(S)-hydroxy 1-oxo 4-/{(phényl méthoxy)carbonyl}amino/butyl/-2-désoxy-N[3]-(méthoxy-carbonyl)D-streptamine.

On dissout 3,47 g de produit obtenu au stade précédent dans un mélange de 22,5 ml de méthanol et 22,5 ml de chlorure de méthylène, glace à 0°C, ajoute 2,25 ml d'hydroxyde de sodium 2N, agite une heure, neutralise par de l'acide chlorhydrique 1N, lave la phase organique à l'eau, extrait la phase aqueuse par du chlorure de méthylène, sèche et évapore. On purifie le produit brut obtenu par chromatographie sur silice en éluant au mélange chloroforme-méthanol (97—3) et recueille 3,116 g du produit cherché sous forme d'un produit amorphe blanc.

Chromatographie en couche mince sur silice (chloroform-méthanol 95/5) Rf = 0,44.

*Stade C*

Acétate de O-/3-(éthoxy carbonyl)méthyl amino 3,4,6-tridésoxy α,D-xylohexopyranosyl (1→6)/-O-/2,6-bis(méthoxy carbonyl amino) 2,3,6-tridésoxy α,D-ribohexopyranosyl (1→4)/ $N^1$-/(2(S)-hydroxy 1-oxo 4-amino)butyl/-2-désoxy $N^3$ (méthoxycarbonyl) D-streptamine.

On dissout, 3,080 g du produit obtenu au stade précédent dans un mélange de 154 ml d'acide acétique et 15,4 ml d'eau, traite par l'hydrogène avec un catalyseur composé de 616 mg de palladium sur charbon à 9,81%. Au bout de 21 heures, on filtre le catalyseur, lave à l'acide acétique, évapore à sec le filtrat, sèche sous vide à chaud et obtient 2,292 g du produit cherché sous forme de poudre amorphe beige.

Chromatographie en couche mince sur silice:chloroform-méthanol-ammoniaque (70—30—2,5). Rf = 0,24.

*Stade D*

O-/2,6-diamino 2,3,6-tridésoxy α,D-ribohexopyranosyl (→4)/-O-/3-(méthylamino) 3,4,6-tridésoxy α,D-xylohexoyranosyl (1→6]$N^1$-(4-amino 2(S)-hydroxy 1-oxo)butyl 2-désoxy D-streptamine.

2,363 g du produit obtenu au stade précédent sont agités dans 11,3 ml d'hydroxyde de potassium 14N. On chauffe à 60°C agite à cette temperature pendant 1h25mn., refroidit à 0°C., neutralise à pH = 3 par de l'acide sulfurique N, purifie par chromatographie sur colonne de résine échangeuse d'ions (commercialisée sous le nom IRP 64 $NH_4+$) en éluant à l'ammoniaque 0,4N, et recueille 1,155 g du produit cherché.

Chromatographie en couche mince sur silice (chloroforme-méthanol-ammoniaque 2—2—1) Rf = 0,18.
SPECTRE DE R.M.N. dans $D_2O$—DCl (60 MHz)

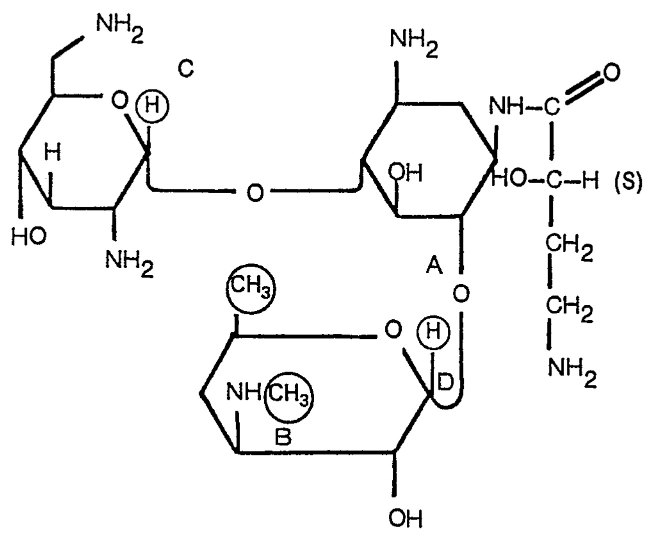

| | | |
|---|---|---|
| A — $CH_3$ | 1,2 p.p.m. | doublet J = 6 |
| B — $CH_3$ | 2,7 p.p.m. | |
| C — H anomère | 5,8 p.p.m. | doublet J = 3,5 |
| D — H anomère | 5,2 p.p.m. | doublet J = 3,0 |

Le produit de départ du stade A peut être obtenu de la façon suivante:

*Stade 1*

2-désoxy 4-O-/2,3,6-tridésoxy 2,6-bis/(méthoxycarbonyl)amino/ α,D-ribohexopyranosyl/ 5,6-O-(1-méthyl)éthylidène $N^1$-$N^3$-bis méthoxycarbonyl D-streptamine.

On dissout 38,5 g de 4'-acétyle 3'-désoxy tétre N-carbométhoxy néamine (préparée selon le brevet française n°2 290 908) dans 155 ml de diméthylformamide, ajoute 20 ml de diméthoxypropane et 200 mg d'acide paratoluène sulfonique.

La solution obtenue est agitée à chaud dans un bain-marie à 90°C. sous atmosphère inerte; après 17 heures de réaction, on ajoute 20 ml de diméthoxypropane et 200 mg d'acide paratoluène sulfonique, après 25 heures de réaction, on ajoute à nouveau 20 ml de diméthoxypropane et 200 mg d'acide paratoluène sulfonique et chauffe à 110°C., laisse à cette température penant 24 heures et

introduit 20 ml de diméthoxypropane et 200 mg d'acide paratoluène sulfonique et laisse en contact pendant 30 heures; On refroidit, ajoute une résine échangeuse d'ions (commercialisée sous le nom DOWEX 1X2 OH $^-$50/100mesh), jusqu'a pH alcalin, filtre, évapore à sec et obtient 45,6 g de produit. On dissout 44,6 g de produit obtenu dans 90 ml de méthanol, refroidit à 0°C et ajoute, goutte à goutte, 90 ml d'hydroxyde de sodium, extrait par l'essence B (eb. 60—80°C) et le chloroforme, sèche et évapore les extraits.

On obtient 33,10 g du produit cherché, sous forme de poudre amorphe beige.
Chromatographie en couche mince sur silice:chloroforme-méthanol 90/10. Rf = 0,40.

*Stade 2*

2-désoxy 4-O-/2,3,6-tridesoxy 2,6-bis/(méthoxycarbonyl)amino/ 4-O-phényl méthyl $\alpha$,D-ribohexopyranosyl 5,6-O-(1-méthyl)éthylidène $N^1$-$N^3$-bis méthoxy carbonyl D-streptamine.

On dissout 25,6 g du produit obtenu au stade précédent dans 85 ml de diméthylformamide, glace et ajoute 17,0 g d'oxyde de baryum, 25,6 g d'hydroxyde de baryum octahydraté et 25,6 ml de bromure de benzyle, laisse réchauffer, agite 24 heures, verse la solution obtenue dans l'eau, extrait au benzène, sèche, évapore.

On obtient 36,0 g de produit brut que l'on purifie par chromatographie sur silice, (éluant:chloroforme-méthanol 98—2) et on recueille 7,614 g du produit cherché pur, sous forme de mousse amorphe beige.
Chromatographie en crouche mince sur silice chloroform-méthanol (95—5) Rf = 0,36.

*Stade 3*

2-désoxy 4-O-/2,3,6-tridésoxy 2,6-bis/(méthoxycarbonyl)amino/ 4-O-phényl méthyl $\alpha$,D-ribohexopyranosyl/ $N^1$-$N^3$ bis méthoxy carbonyl D-streptamine.

On dissout 7,585 g de produit obtenu au strade précédent dans 25 ml d'une solution aqueuse d'acide acétique chauffe modérément sous agitation pendant 1 heure, ajoute de l'eau, refroidit quelques heures à 0°C. les cristaux obtenus que l'on essore, lave et sèche.

On obtient 5,204 g du produit cherché sous forme d'aiguilles blanches. PF = 251°C.
Chromatographie en couche mince sur silice chloroform-méthanol 90/10 RF = 0,29.

*Stade 4*

1,6 N-O-(carbonyl) 2-désoxy 4-O-/2,3,6 tridésoxy 2,6-bis/(méthoxycarbonyl)amino/ 4-O-phényl méthyl $\alpha$,D-ribohexopyranosyl/$N^3$-méthoxy carbonyl D-streptamine.

5,000 g du produit obtenu au stade précédent et 0,9 g provenant d'une préparation antérieure sont dissouts dans 118 ml de dimethylformamide, refroidis à 0°C., additionnés de 5,90 ml d'alcool tert-butylique et de 5,3 g de tert-butylate de sodium; on dilue au bout de 16 heures par du diméthylformamide, et neutralise par 5,9 ml d'acide acétique dans le même volume de diméthylformamide, verse la solution obtenue dans l'eau, extrait au chloroforme, sèche et évapore les extraits.

On obtient 5,719 g du produit cherché, sous forme de produit amorphe blanc.
Chromatographie en couche mince sur silice chloroforme-méthanol 90/10. Rf = 0,18.

*Stade 5*

2-désoxy 4-O-/2,3,6-tridésoxy 2,6-bis/(méthoxycarbonyl)amino/ 4-O-phényl méthyl $\alpha$,D-ribohexopyranosyl/$N^3$-méthoxy carbonyl D-streptamine.

5,600 g du produit obtenu au stade précédent et 1,038 g provenant d'une préparation antérieure sont mis en suspension dans 89,6 ml d'eau oxygénée à 20 volumes et 66,3 ml d'hydroxyde de sodium N sous agitation, après 4 heures, on glace, neutralise l'excès d'eau oxygénée par du thiosulfate de sodium, acidifie à pH = 7 à l'acide sulfurique, évapore à sec, extrait le résidu avec un mélange chloroforme-méthanol, filtre et lave les sels minéraux, évapore le filtrat, purifie par chromatographie sur silice en éluant au chloroforme-méthanol-ammoniaque 70—30—2. et obtient 2,810 g du produit pur cherché.
Chromatographie en couche mince sur silice:chloroforme-méthanol-ammoniaque 70—30—2. Rf = 0,29.

*Stade 6*

2-désoxy 4-O-/2,3,6-tridesoxy 2,6-bis/(méthoxy carbonyl)amino/ 4-O-phényl méthyl $\alpha$,D-ribohexopyranosyl/ $N^1$-{2(S)-acétyloxy 1-oxo 4//(phényl méthoxy)carbonyl/amino/butyl}-$N^3$-méthoxy carbonyl D-streptamine.

1,785 g d'acide (S) $\alpha$-acétyloxy $\gamma$-N-benzyloxy carbonylamido n-butyrique sont dissouts dans 34 ml de tétrahydrofuranne anhydre, on ajoute 0,84 ml de triéthylamine, 0,516 ml de chloroformiate d'éthyle, agite pendant 1 heure, ajoute 2,760g du produit obtenu ai strade précédent en solution dans 45 ml de diméthylformamide, agite 1h15, ajoute de l'eau (100 ml), verse lentement sous agitation dans 1,6 l d'eau, glace 2 heures à 0°C., essore, lave et sèche le précipité.

On obtient 3,759 g du produit pur cherché.

Chromatographie en couche mince sur silice:chloroforme-méthanol 90—10. Rf = 0,26.

L'acide (S)$\alpha$-acétyloxy $\gamma$-N-benzyloxy carbonylamido n-butyrique utilisé au départ du stade 6, peut être obtenu selon le procédé suivant:

On agite 3 g d'acide (S)$\alpha$-hydroxy $\gamma$-carbonylbenzyl aminobutyrique (préparé selon le brevet français 2 145 649) en solution dans 3 ml de pyridine et 10 ml de chlorure de méthylène avec 2 ml d'acide acétique; au bout de 3 h. on dilue au chlorure de méthylène, lave, sèche, évapore à sec et obtient 3,45 g du produit cherché sous forme d'huile incolore, servant telle quelle dans le stade 6.

Exemple 2

Sulfate de O-/2,6-diamino 2,3,6-tridésoxy $\alpha$,D-ribohexopyranosyl (1→4)/-O-/3-méthylamino 3,4,6-tridésoxy $\alpha$,D-xylohexopyranosyl (1→6)/ N[1]-(4-amino 2(S)-hydroxy 1-oxo) butyl 2-désoxy D-streptamine.

On dissout dans l'eau 1,00 g du produit obtenu à l'exemple précédent, acidifie à pH = 3 par de l'acide sulfurique, concentre, filtre, reconcentre, verse sous agitation dans le méthanol, glace en agitant quelques heures, essore, lave et sèche le précipité.

On obtient 1,4 g sulfate sous forme d'une poudre blanche amorphe.

Chromatographie en couche mince sur silice:chloroforme-méthanol-ammoniaque 2/2/1: Rf = 0,19.

SPECTRE R.M.N. ($D_2O$) (60MHz)

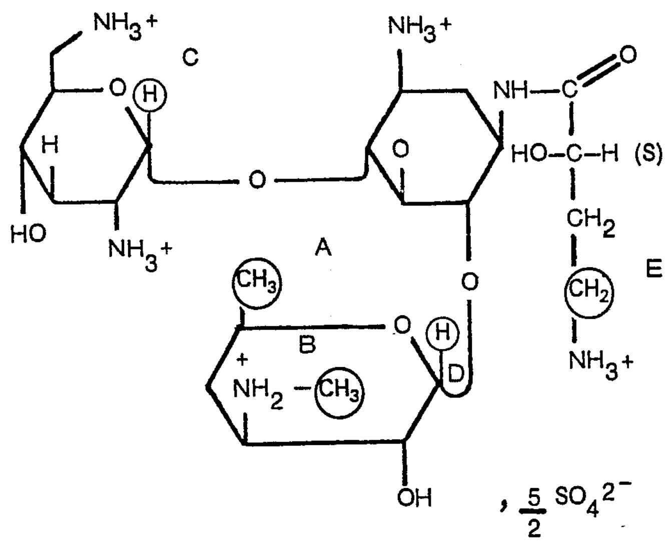

A — 1,22 p.p.m. doublet J = 6,5

B — 2,7 p.p.m.

C — H anomère 5,9 p.p.m. doublet J = 3,5

D — H anomère 5,2 p.p.m. doublet J = 3,5

E — 3,2 p.p.m. triplet J = 7

Exemple 3

1'O-/2,6-diamino 2,4,6-tridésoxy $\alpha$,D-xylohexopyranosyl (1→4)/-O-/3-méthylamino 3,4,6-tridésoxy $\alpha$,D-xylohexoypyranosyl (1→6)/-N[1]-/4-amino 2(S)-hydroxy 1-oxo butyl/2 désoxy D-streptamine.

*Stade A*

1'O-/3-(éthoxycarbonyl méthylamino) 2-O-phénylméthyl 3,4,6-tridésoxy $\alpha$,D-xylohexopyranosyl (1→6)-O-/2,4,6-tridésoxy 2,6-bis/(méthoxy carbonyl)amino/ 3-O-phényl méthyl $\alpha$,D-xylohexopyranosyl (1→4)/ N[1]-/2(S)-acétyloxy 1-oxo 4-//(phényl méthoxy)carbonyl/amino/butyl/2-désoxy N[3]-méthoxycarbonyl D-streptamine.

On dissout dans 64,5 ml de chlorure de methylène anhydre saturé d'acide bromhydrique, 1,42 g de 1-O-acétyl 2-O-benzyl 3-(N-carbéthoxy N-méthyl)amino 3,4,6-tridésoxy D-xylohexopyranose, agite 45 minutes à température ambiante, ajoute du toluène, évapore à sec; on dissout le résidu sec dans 52 ml de chlorure de méthylène, ajoute 815 mg de bromure de tétraéthyl ammonium, 0,7 ml de diisopropyléthylamine, 2,195 g de N[1]/2(S)-acétyloxy) 1-oxo 4//(phényl méthoxy)carbonyl/amino/butyl/ 2-désoxy 4-O-/2,4,6-tridésoxy 2,6bis-(méthoxy carbonyl)amino 3-O-phényl méthyl $\alpha$,D-

xylohexopyranosyl]N³-méthoxy carbonyl-D-streptamine, chauffe au reflux, au bout de 4h30 rajoute 0,5 équivalent de dérivé bromé comme préparé ci-dessus, 0,5 équivalent de bromure de tétraéthyl ammonium et 0,5 équivalent de diisopropyléthylamine et effectue le même rajout au bout de 21 heures.

Après 28 heures de chauffage, on dilue au chlorure de méthylène, lave à l'eau et évapore à sec.

On obtient 4,428 g produit brut cherché, qui, purifié par chromatographie sur silice donne 2,107 g de produit pur.

Chromatographie sur couche mince sur silice:chloroforme-acétone 60/40: Rf = 0,25.

*Stade B*

O-/3,4,6-tridésoxy 3-(éthoxycarbonyl méthylamino) 2-O-phényl méthyl $\alpha$,D-xylohexopyranosyl (1→6)/-O-/2,4,6-tridésoxy 2,6-bis (méthoxycarbonyl)-amino 3-O-phenyl méthyl $\alpha$,D-xylohexopyranosyl (1→4)/N¹-/2(S)-hydroxy 1-oxo-4-//(phényl méthoxy)carbonyl/amino/butyl/ 2-désoxy N³-(méthoxycarbonyl) D-streptamine.

On dissout 1,670 g du produit obtenu au stade précédent dans 11 ml de chlorure de méthylène et 11 ml de méthanol, glace, ajoute 1,1 ml de soude 2N, agite 1 heure à 0°C., dilue à l'eau glacée, neutralise à pH = 7 à l'acide chlorhydrique, extrait par le chlorure de méthylène, lave et évapore les extraits.

On obtient 1,556 g du produit cherché brut que l'on purifie par chromatographie sur silice et recueille 1,388 g de produit pur, sous forme d'une mousse beige.

*Stade C*

Acétate de O-/3,4,6-tridésoxy 3-(éthoxycarbonyl methylamino) $\alpha$,D-xylohexopyranosyl (1→6)/-O-/2,4,6-tridésoxy 2,6-bis (méthoxycarbonyl)amino $\alpha$,D-xylohexopyranosyl (1→4)/-N¹-/(4-amino 2(S)-hydroxy 1-oxo)butyl/2-désoxy N³-(méthoxycarbonyl) D-streptamine.

1,365 g du produit obtenu au stade précédent sont traités par l'hydrogène avec un catalyseur composé de 275 mg de palladium sur charbon, dans un mélange de 68 ml d'acide acétique et 6,8 ml d'eau. Au bout de 22 heures, on filtre le catalyseur, lave à l'acide acétique, évapore à sec le filtrat, sèche sous vide et obtient 1,002 g du produit cherché sous forme d'un solide blanc.

Chromatographie en couche mince sur silice:chloroforme-méthanol-ammoniaque 70/30/2,5. Rf = 0,17.

*Stade D*

O-/2,6-diamino 2,4,6-tridésoxy $\alpha$,D-xylohexopyranosyl (1→4)/-O-/3-(méthylamino) 3,4,6-tridésoxy $\alpha$,D-xylohexopyranosyl (1→6)/-N¹-/4-amino 2(S)-hydroxy 1-oxo butyl/2-désoxy D-streptamine.

On verse 0,980 g du produit obtenu au stade précédent finement broyé dans 4,9 ml de potasse 14N sous agitation, dans un bain à 65°C, au boute de 2h15, on refroidit à 0°C, acidifie à pH = 3 par de l'acide sulfurique et obtient une solution jaunâtre que l'on dépose puis élue à l'ammoniaque 0,4N, sur une colonne de résine, on obtient 0,467 g du produit cherché sous forme d'une poudre amorphe blanche.

Chromatographie en couche mince sur silice:chloroforme-méthanol-ammoniaque 2/2/1. Rf = 0,14.

SPECTRE DE R.M.N. (dans $D_2O$) (60MH$_z$)

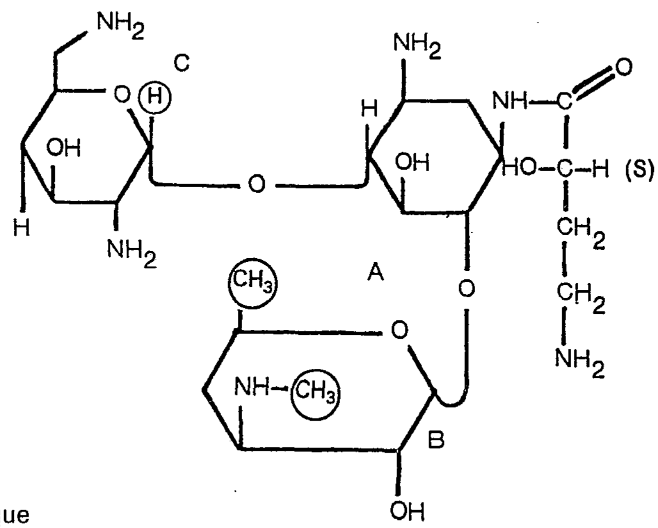

en milieu chlorhydrique

A : $CH_3$ — 1,85 p.p.m. doublet J = 6 cps

B : $CH_3$ — 4,1 p.p.m.

C : H anomère — 9,04 p.p.m. doublet J = 3 cps

Le produit de départ du stade A peut être obtenu de la façon suivante:

*Stade 1*

5,6-O-cyclohexylidène 4'-O-tosyl tétra-N-carbométhoxy néamine.

On fait réagir dans de la pyridine 795 g de chlorure de tosyle avec 1590 g de 5,6-cyclohexylidène tétra-N-carbométhoxy néamine (J. of Antibiotics, 1971, 711), maintient la température 1 h à 2°C., laisse revenir à température ambiante, agite pendant 40 h, ajoute de formiate de potassium (530g) par petites fractions puis agite 1 h, redissout l'extrait gommeux dans 2,5 l d'eau et 2,5 l de chlorure de méthylène, lave la phase organique à l'eau, l'extrait au chlorure de méthylène, sèche puis distille les phases organiques, triture trois fois la résine obtenue à l'éther isopropylique, concentre sous vide à 80°C, redissout le résidu gommeux dans 3 l de chlorure de méthylène, cristallise, lave les cristaux, sèche. On obtient 560 g du composé cherché.

$/\alpha/_D$ 0,5% $CHCl_3$ : +19°±3°.

*Stade 2*

5,6-O-cyclohexylidène 2-désoxy 4-O-/2,4,6-tridésoxy 4-iodo 2,6-bis (méthoxycarbonyl)amino $\alpha$,D-xylohexopyranosyl $N^1$,$N^3$-bis (méthoxycarbonyl) D-streptamine.

On chauffe à 115°C, 30,3 g d'iodure de sodium dans 130ml de diméthylformamide, ajoute 13,3 g du produit obtenu au stade précédent, au bout de 2h30, laisse refroidir, reprend la solution à l'eau, extrait cinq fois par du chlorure de méthylène, sèche et évapore les extraits. On obtient 7,843 g d'huile du produit cherché qui se concrétise en mousse.

Chromatographie en couche mince sur silice:chloroforme-méthanol 90—10. Rf = 0,45.

*Stade 3*

5,6-O-cyclohexylidène 2-désoxy 4-O/2,4,6-tridésoxy 2,6-bis(méthoxycarbonyl)amino $\alpha$,D-xylohexopyranosyl/$N^1$,$N^3$-bis(méthoxycarbonyl) D-streptamine.

On ajoute dans une suspension de 35 ml de Nickel de Raney dans 150 ml d'éthanol, 7,84 g du produit obtenu au stade précédent, agite pendant l heure, filtre, évapore le filtrat. On obtient 5,17 g du produit cherché sous forme d'une mousse blanche qui, purifiée par chromatographie sur silice, permet de recueillir 4,506 g produit pur.

Chromatographie en couche mince sure silice:chloroforme-méthanol 90—10. Rf = 0,40.

*Stade 4*

5,6-O-cyclohexylidène 2-désoxy 4-O-/2,4,6-tridésoxy 2,6-bis(méthoxycarbonyl)amino 3-O-phénylméthyl $\alpha$,D-xylohexopyranosyl/ $N^1$,$N^3$-bis (méthoxycarbonyl) D-streptamine.

On dissout 13,355 g de produit tel qu'obtenu au stade précédent dans 53,5 ml de diméthylformamide, glace la solution, ajoute 13,355 g de baryte, 13,355 g d'oxyde de baryum et 13,355 g de bromure, de benzyle, agite pendant 20 heures à 0°C, laisse réchauffer, agite encore 5 heures, verse dans l'eau, extrait à cinq reprises au chloroforme, sèche et évapore les extraits, purifie à deux reprises par chromatographie sur silice le produit brut obtenu et obtient 9,279 g du produit pur cherché.

Chromatographie en couche mince sur silice:chloroforme-méthanol 95—5. Rf = 0,44.

*Stade 5*

2-désoxy 4-O-/2,4,6-tridésoxy 2,6-bis (méthoxycarbonyl)amino 3-O-phényl méthyl $\alpha$,D-xylohexopyranosyl/$N^1$,$N^3$-bis(méthoxycarbonyl) D-streptamine.

On dissout 10 g du produit tel qu'obtenu au stade précédent dans 30 ml d'acide acétique à 80%, chauffe à 60°C et agite 1 heure 15, refroidit, évapore à sec.

On obtient 7,615 g du produit cherché.

Chromatographie en couche mince sur silice:chloroforme-méthanol 90—10. Rf = 0,25.

*Stade 6*

1,6-N,O-(carbonyl) 2-désoxy 4-O-/2,4,6-tridésoxy 2,6-bis(méthoxycarbonyl)amino 3-O-phénylméthyl $\alpha$,D-xylohexopyranosyl/$N^3$-méthoxycarbonyl D-streptamine.

On dissout 7,6 g du produit obtenu au stade précédent dans 150 ml de diméthylformamide, ajoute 7,6 ml de tert-butanol et refroidit dans un bain de glace, agite ajoute 6,65 g de tert-butylate de potassium, agite pendant 18 h, dilue par du diméthylformamide, acidifie par 7,6 ml d'acide acétique et 7,6 ml de diméthylformamide, verse dans de l'eau, extrait au chlorure de méthylène et lave à l'eau, sèche et évapore les extraits.

On obtient 7,145 g du produit cherché.
Chromatographie en couche mince sur silice:chloroforme-méthanol 90/10 2 passages: Rf = 0,42.

*Stade 7*

2-désoxy 4-O-/2,4,6-tridésoxy 2,6-bis (méthoxycarbonyl)amino 3-O-phényl méthyl α,D-xylohexopyranosyl/ $N^3$-méthoxycarbonyl D-streptamine.

On dissout 6,170 g du carbamate cyclique obtenu au stade précédent dans 10 ml de chloroforme, ouvre le cycle carbamate par 45 ml d'eau oxygénée et 60 ml de soude, agite 1 heure, glace, neutralise l'excès d'eau oxygénée au thiosulfate de sodium, acidifie à pH = 5 avec de l'acide sulfurique, extrait au chloroforme le produit cherché, évapore, purifie à deux reprises par chromatographie sur silice et obtient 1,718 g du produit cherché sous form de mousse jaunâtre.
Chromatographie en couche mince sur silice:chloroforme-méthanol-ammoniaque 30/20/1: Rf = 0,18.

*Stade 8*

$N^1$-/2(S)-acétyloxy 1-oxo 4-//(phénylméthoxy)carbonyl/amino/butyl/ 2-désoxy 4-O-/2,4,6-tridésoxy 2,6-bis(méthoxycarbonyl)amino 3-O-phényl méthyl α,D-xylohexopyranosyl/ $N^3$-méthoxycarbonyl D-streptamine.

On dissout 880 mg d'acide (S) α-acétoxy γ-N-benzyloxy carbonylamido n-butyrique dans du tétrahydrofuranne anhydre, ajoute 0,413 ml de triéthylamine, 0,256 ml de chloroformiate d'éthyle, agite, introduit 1,362 g du produit obtenu au stade précédent en solution dans du diméthylformamide, agite 2h, ajoute de l'eau, glace quelques heures, essore, lave et sèche.

On obtient 1,748 g du produit cherché sous forme d'aiguilles blanches.
PF = 199°C.
Chromatographie en couche mince sur silice:chloroforme-méthanol 90/10: Rf = 0,34.

Exemple 4

Sulfate de O-/2,6-diamino 2,4,6-tridésoxy α,D-xylohexopyranosyl (1→4)/-O-/3-(méthylamino) 3,4,6-tridésoxy α,D-xylohexopyranosyl (1→6)/-$N^1$-/4-amino 2(S)-hydroxy 1-oxo butyl/ 2-désoxy D-streptamine.

On dissout 350 mg produit obtenu à l'exemple 3 dans 20 ml d'eau, neutralise à pH = 3 par de l'acide sulfurique, concentre, filtre, reconcentre, verse sous agitation dans du méthanol, glace, filtre.

On obtient 481 mg du produit cherché sous forme de produit amorphe blanc.
Chromatographie en couche mince sur silice:chloroforme-méthanol-ammoniaque: 2/2/1: Rf = 0,14.
SPECTRE R.M.N. en $D_2O$
60 Mhz.

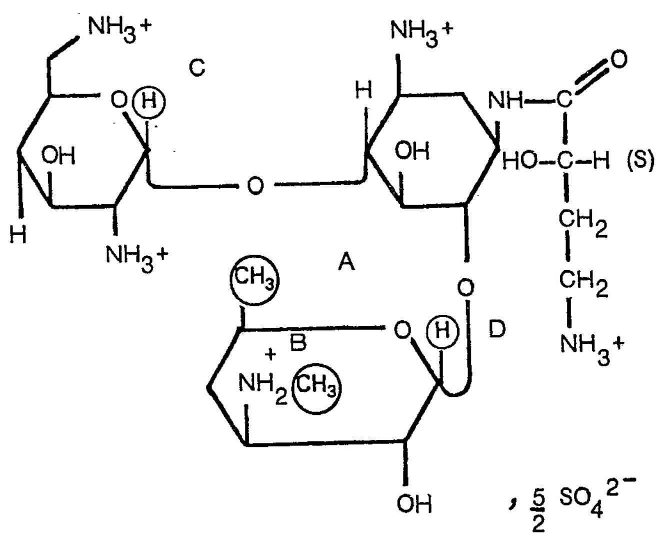

A — 1,2 p.p.m. doublet J = 6

B — 2,6 p.p.m.

C — H anomère 6,1 p.p.m. doublet J = 3,5

D — H anomère 5,2 p.p.m. doublet J = 3,5

Exemple 5

O-/2,6-diamino 2,3,4,6-tétradésoxy $\alpha$,D-érythrohexopyranosyl (1→4)/-O-/3,4,6-tridésoxy 3-(méthylamino) $\alpha$,D-xylohexopyranosyl (1→6)/ $N^1$-(4-amino 2(S)-hydroxy 1-oxo)butyl 2-désoxy D-streptamine.

*Stade A*

O-/2,6-diméthoxy carbonylamino 2,3,4,6-tétradésoxy $\alpha$,D-érythrohexopyranosyl (1→4)/-O-/3,4,6-tridésoxy 3-(éthoxy carbonyl)méthylamino 2-O-phénylméthyl $\alpha$,D-xylohexopyranosyl (1→6)/ $N^1$-/2(S)-acétyloxy 1-oxo 4-//(phénylméthoxy)carbonyl/amino/butyl/ 2-désoxy $N^3$-(méthoxycarbonyl) D-streptamine.

On dissout dans du chlorure de méthylène saturé d'acide bromhydrique, 4,14 g de 1-O-acétyl 2-O-benzyl 3-(N-carbéthoxy N-méthyl) amino 3,4,6-tridésoxy D-xylohexopyranose, agite, évapore à sec, reprend au chlorure de méthylène, ajoute 1,92cm3 de diisopropyl éthylamine, 2,38 g de bromure de tétraéthylammonium et 45 g de 2-désoxy 4-O-/2,3,4,6-tétradésoxy 2,6-bis(méthoxycarbonyl) amino/ $\alpha$,D-érythrohexopyranosyl $N^1$-/2(S)-acétyloxy 1-oxo 4//(phénylméthoxy) carbonyl/amino/butyl/ 2-désoxy $N_3$-méthoxycarbonyl D-streptamine, chauffe au reflux, après 21h30 refroidit, dilue au chlorure de méthylène, lave deux fois à l'eau, sèche la phase organique, filtre, évapore à sec, purifie par chromatographie sur colonne de silice et obtient 32,35 g de produit cherché pur.
Chromatographie en couche mince sur silice:chloroforme-acétone 7/3: Rf = 0,16.

*Stade B*

O-/3-(méthoxycarbonyl)méthyl amino 2-O-phényl méthyl 3,4,6-tridésoxy $\alpha$,D-xylohexopyranosyl (1→6)/-O-/2,6-bis (méthoxycarbonyl) amino 2,3,4,6-tétradésoxy $\alpha$,D-érythrohexopyranosyl (1→4) $N^1$-/2(S)-hydroxy 1-oxo 4-//(phénylméthoxy)carbonyl/amino/butyl/ 2-désoxy $N^3$-méthoxycarbonyl D-streptamine.

On dissout 15,2 g de composé obtenu à l'étape précédente dans un mélange de chlorure de méthylène et de méthanol, glace à 0°C, ajoute de la soude 2N, agite 1 heure, neutralise à l'acide chlorhydrique, extrait au chlorure de méthylène, lave, sèche, filtre et évapore à sec.

On obtient 14,6 g du produit cherché.
Chromatographie en couche mince sure silice:chloroforme-méthanol 65/35: Rf = 0,12.

*Stade C*

Acétate de O-/3-(éthoxy carbonyl) méthyl amino 3,4,6-tridésoxy $\alpha$,D-xylohexopyranosyl (1→6)/-O-/2,6-bis(méthoxycarbonyl) amino 2,3,4,6-tétradésoxy $\alpha$,D-érythrohexopyranosyl (1→4)/-$N^1$-/(2(S)-hydroxy 1-oxo 4-amino)butyl/ 2-désoxy $N^3$-méthoxycarbonyl D-streptamine.

On dissout 14,6 g du produit obtenu au stade précédent dans un mélange de 10 parties à 1 d'acide acétique et d'eau, traite par l'hydrogène en présence d'un catalyseur composé de 3,1 g de palladium sur charbon à 10%, au bout de 16 h filtre, lave, évapore à sec le filtrat, sèche et obtient 11,7 g du produit cherché.
Chromatographie en couche mince sur silice:chloroforme-méthanol-ammoniaque 80/20/2. Rf = 0,33.

*Stade D*

O-/2,6-diamino 2,3,4,6-tétradésoxy $\alpha$,D-érythrohexopyranosyl (1→4)/-O-/3,4,6-tridésoxy 3-méthylamino $\alpha$,D-xylohexopyranosyl (1→6)/$N^1$-/4-amino 2(S)-hydroxy 1-oxo-butyl/ 2-désoxy D-streptamine.

On agite dans 8 ml d'hydroxyde de potassium 14N, 11,7 g du produit obtenu au stade précédent, chauffe, agite pendant 9 heures, refroidit et neutralise à pH = 3 par de l'acide sulfurique N, purifie par chromatographie sur colonne de résine; par élution à l'ammoniaque, on recueille 10,35 g du produit cherché.
Chromatographie en couche mince sur silice:chloroforme-méthanol-ammoniaque 2/2/1: Rf = 0,27.

SPECTRE R.M.N. ($D_2O$, 60 MHz)

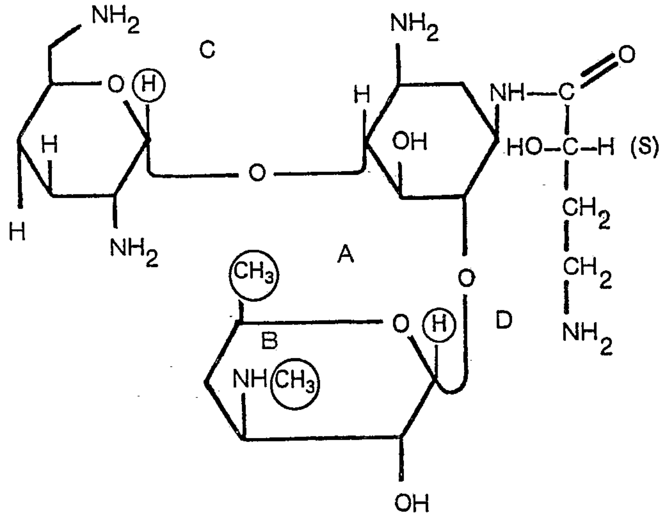

A — $CH_3$ 1,2 p.p.m. doublet J = 6
B — $CH_3$ 2,7 p.p.m.
C — H anomère 5,8 p.p.m. doublet J = 3,5
D — H anomère 5,1 p.p.m. doublet J = 3

Le produit de départ du stade A peut étre obtenu de la façon suivante:

*Stade 1*

1,6-N-O-(carbonyl) 2-désoxy 4-O-/2,3,4,6-tétradésoxy 2,6-bis/(méthoxycarbonyl)amino/ $\alpha$,D-érythrohexopyranosyl/N³-méthoxycarbonyl D-streptamine.

On dissout 20 g de 3'4'-didésoxy tétra N-carbométhoxy néamine (préparée selon le brevet français n° 2 263 745) dans 180 cm3 de diméthylformamide en présence d'alcool tert-butylique et de tert-butylate de potassium, agite, refroidit, introduit lentement 5,5 g d'hydrure de sodium à 50% sous huile, au bout de 21 h, détruit l'excès d'hydrure par de l'acide acétique, ajoute de l'eau, évapore, reprend le résidu dans un mélange de chloroforme et d'éthanol, filtre, évapore le filtrat, obtient 30 g d'un sirop que l'on utilise tel quel pour l'étape suivante.

*Stade 2*

2-désoxy 4-O-/2,3,4,6-tetradésoxy 2,6-bis/(méthoxycarbonyl)amino/ $\alpha$,D-érythrohexopyranosyl/ N³-méthoxycarbonyl D-streptamine.

On ajoute au sirop obtenu à l'étape précédente de la soude normale et de l'eau oxygénée à 20 volumes en volumes égaux agite, élimine après 5 h l'excès de péroxyde par du thiosulfate, de sodium, amène à pH=7 par de l'acide sulfurique, concentre, reprend par un mélange de 9 parties à 1 de chloroforme et de méthanol, filtre, évapore à sec, purifie par chromatographie sur colonne de silice et obtient 8,1 g du produit cherché.
Chromatographie en couche mince sur silice: chloroforme-méthanol-ammoniaque 80/20/1. Rf = 0,12.

*Stade 3*

2-désoxy 4-O-/2,3,4,6-tétradésoxy 2,6-bis/(méthoxycarbonyl)amino/ $\alpha$,D-xylohexopyranosyl N¹-/2(S)-acétyloxy 1-oxo 4-//(phényl méthoxy)carbonyl/amino/butyl/ N³-méthoxycarbonyl D-streptamine.

On dissout 2,95 g d'acide (S) $\alpha$-acétyloxy $\gamma$-N-benzyloxy carbonylamido N-butyrique dans du tétrahydrofuranne, ajoute 1,39 cm3 de triéthanolamine, 0,9 cm3 de chloroformiate d'éthyle et 3,715 g du produit obtenu à l'étape précédente dissouts dans du diméthylformamide, agite, au bout d'une heure verse dans de l'eau glacée, extrait au chloroforme, lave à l'eau, sèche, filtre, évapore à sec, purifie par chromatographie sur colonne de silice.

On obtient 4,75 g du produit pur cherché.
Chromatographie en couche mince sur silice: chloroforme-méthnol 95/5. Rf=0,15.

Exemple 6

Sulfate de O-/2,6-diamino 2,3,4,6-tétradésoxy $\alpha$,D-érythrohexopyranosyl (1→4)/-O-/3,4,6-tridésoxy 3-méthylamino $\alpha$,D-xylohexopyranosyl (1→6)/N¹-/4-amino 2(S)-hydroxy 1-oxo butyl/ 2-désoxy D-streptamine.

On dissout dans l'eau 10,0 g du produit de l'exemple 5, acidifie à pH=3 par de l'acide sulfurique 0,1N, concentre, filtre, reconcentre, cristallise dans le méthanol à 0°C, essore, lave et séche.

On obtient 14 g du produit cherché.

Chromatographie en couche mince sur silice, chloroforme-méthnol-ammoniaque: 2/2/1. Rf=0,25.

SPECTRE R.M.N. en $D_2O$ (60 Mhz)

A — $CH_3$ 1,2 p.p.m. doublet J = 6
B — $CH_3$ 2,7 p.p.m.
C — H anomère 5,9 p.p.m. doublet J = 4
D — H anomère 5,1 p.p.m. doublet J = 9,5

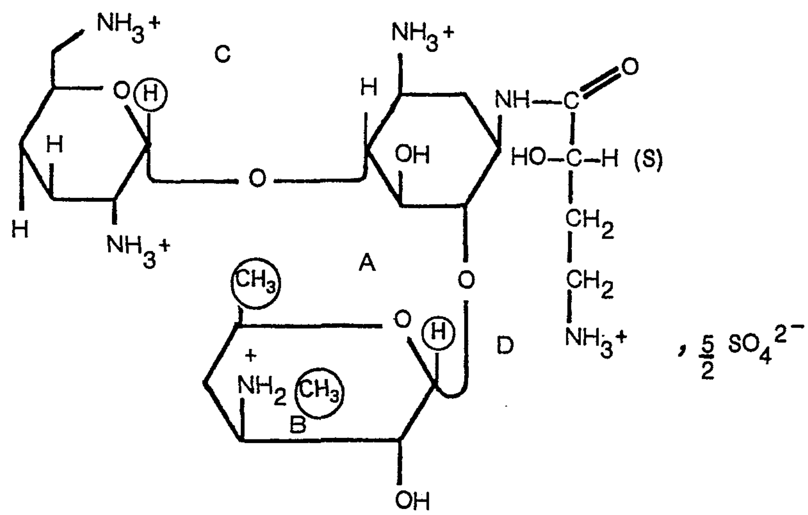

Exemple 7

On a réalisé une préparation pour injection de formule:

— Produit décrit à l'exemple 2 50 mg

— Excipient aqueux stérile 1 cm3

Exemple 8
ETUDE PHARMACOLOGIQUE DU PRODUIT DE L'EXEMPLE 2
Activité antibactérienne in vitro

L'activité antibactérienne a été mesurée in vitro par la méthode des dilutions en milieu liquide.

On prépare une série de tubes dans lesquels on répartit une même quantité de milieu nutritif. On distribue des quantités croissantes de l'antibiotique étudié puis chaque tube est ensemencé avec une souche bactérienne. Après une incubation de dix-huit, vingt-quatre ou quarante-huit heures à 1'étuve à 37°C., l'inhibition de la croissance bactérienne est appréciée par transillumination, ce qui permet de déterminer les concentrations minimales inhibitrices (CMI) du produit, exprimées ici en $\mu$g/cm3 de base.

On a obtenu les résultats suivants:

PRODUIT DE L'EXEMPLE 2

| SOUCHES | Lecure après | | |
|---|---|---|---|
| | 18 h. | 24 h. | 48 h. |
| Staphylococcus aureus ATCC 6538 P.S. | 0,2 | 0,5 | 1 |
| Staphylococcus aureus U.C. 1128 P.R. | 0,2–0,5 | 0,5 | 1 |
| Staphylococcus aureus Exp. N° 54146 | 0,2 | 1 | 2 |
| Staphylococcus aureus Co 15 R cephalexine | 0,1 | 0,2 | 0,5 |
| Streptococcus pyogènes A 561 | 0,5 | 0,5 | 1 |
| Bacillus subtilis ATCC 6633 | 10 | 20 | 40 |
| Escherichia Coli ST ATCC 9637 | 0,5 | 0,5 | 1 |
| Escherichia Coli RT ATCC 11303 | 0,5 | 0,5 | 1 |
| Escherichia Coli Exp. T $O_{26}B_6$ | 0,5 | 1 | 1 |
| Escherichia Coli RG R 55 123 D | 0,5 | 0,5 | 0,5 |
| Klebsiella pneumoniae Exp. 52145 | 0,1 | 0,1 | 0,1 |
| Klebsiella pneumoniae 2536 R | 0,2 | 0,2 | 0,2 |
| Proteus mir. (indol–) A 235 | 2 | 2 | 3 |
| Proteus vulgaris (indol+) A 232 | 2 | 3 | 3 |
| Enterobacter cloacae 681 | 0,1 | 0,2 | 0,2 |
| Providencia Du 48 | 3 | 5 | 5 |
| Pseudomonas 3935 Exp. SG | 1 | 1 | 2 |
| Pseudomonas 8951 RGT | 3 | 20 | >40 |
| Serratia RG 2532 | 0,5 | 0,5 | 1 |

EXEMPLE 9: ETUDE PHARMACOLOGIQUE DU PRODUIT DE L'EXEMPLE 4

Activité antibactérienne in vitro mesurée dans les mêmes conditions que dans l'exemple 8

| SOUCHES | Lecture après | | |
|---|---|---|---|
| | 18 h. | 24 h. | 48 h. |
| Staphylococcus aureus ATCC 6538 P.S. | 1 | 1 | 2 |
| Staphylococcus aureus U.C. 1128 P.R. | 0,2–0,5 | 0,5 | 1 |
| Staphylococcus aureus Exp. N° 54146 | 0,5 | 1 | 2 |
| Staphylococcus aureus Co 15 R cephalexine | 0,5 | 0,5 | 2 |
| Streptococcus pyogènes A 561 | 0,5 | 1 | 1,5 |
| Bacillus subtilis ATCC 6633 | 0,1 | 0,2 | 0,5 |
| Escherichia Coli ST ATCC 9637 | 1 | 2 | 2 |
| Escherichia Coli RT ATCC 11303 | 0,5 | 0,5 | 2 |
| Escherichia Coli Exp. T $O_{26}B_6$ | 1 | 1 | 2 |
| Escherichia Coli RG R 55 123 D | 0,5 | 0,5 | 1 |
| Klebsiella pneumoniae Exp. 52145 | 0,2 | 0,2 | 0,5 |
| Klebsiella pneumoniae 2536 R | 0,2 | 0,2 | 0,5 |
| Proteus mir. (indol–) A 235 | 3 | 5 | 5 |
| Proteus vulgaris (indol+) A 232 | 3 | 10 | 20 |
| Enterobacter cloacae 681 | 0,5 | 0,5 | 0,5 |
| Providencia Du 48 | 20 | 40 | 40 |
| Pseudomonas 3935 Exp. SG | 2 | 2 | 5 |
| Pseudomonas 8951 RGT | 1 | 1 | 3 |
| Serratia RG 2532 | 1 | 1 | 2 |

EXEMPLE 10: ETUDE PHARMACOLOGIQUE DU PRODUIT DE L'EXEMPLE 6

Activité antibactérienne in vitro mesurée dans les mêmes conditions que dans l'exemple 8

| SOUCHES | Lecture après | | |
|---|---|---|---|
| | 18 h. | 24 h. | 48 h. |
| Staphylococcus aureus ATCC 6538 P.S. | 0,5 | 0,5 | 1 |
| Staphylococcus aureus U.C. 1128 P.R. | 0,5 | 0,5 | 1 |
| Staphylococcus aureus Exp. N° 54146 | 0,2 | 2 | 5 |
| Staphylococcus aureus Co 15 R cephalexine | 0,1 | 0,2 | 0,5 |
| Streptococcus pyogènes A 561 | 1 | 2 | 3 |
| Bacillus subtilis ATCC 6633 | 0,05 | 0,05 | 0,2 |
| Escherichia Coli ST ATCC 9637 | 1 | 1 | 1 |
| Escherichia Coli RT ATCC 11303 | 0,5 | 0,5 | 1 |
| Escherichia Coli Exp. T $O_{26}B_6$ | 1 | 1 | 1 |
| Escherichia Coli RG R 55 123 D | 0,5 | 0,5 | 0,5 |
| Klebsiella pneumoniae Exp. 52145 | 0,1 | 0,1 | 0,1 |
| Klebsiella pneumoniae 2536 R | 0,2 | 0,2 | 0,5 |
| Proteus mir. (indol-) A 235 | 2 | 3 | 5 |
| Proteus vulgaris (indol+) A 232 | 1 | 2 | 2 |
| Enterobacter cloacae 681 | 0,2 | 0,2 | 0,2 |
| Providencia Du 48 | 3 | 5 | 20 |
| Pseudomonas 3935 Exp. SG | 1 | 1 | 2 |
| Pseudomonas 8951 RGT | 1 | 2 | 2 |
| Serratia RG 2532 | 0,5 | 0,5 | 1 |

**Revendications**

1. Nouveaux aminoglycosides dérivés de la désoxy streptamine, ainsi que leurs sels d'addition avec les acides minéraux ou organiques, caractérisés en ce qu'ils répondent à la formule générale I:

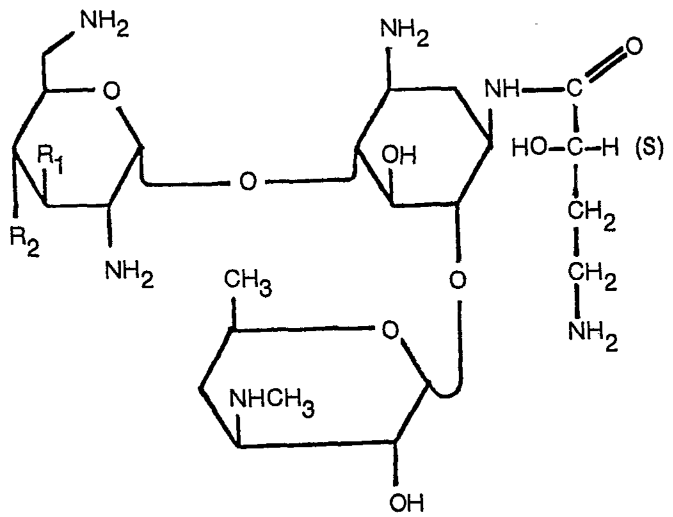

I

dans laquelle les substituants $R_1$ et $R_2$ représentent un atome d'hydrogène ou un groupement hydroxyle, l'un au moins de ces substituants n'étant pas un groupement hydroxyle.

2. L' O-/2,6-diamino 2,3,6-tridésoxy $\alpha$,D-ribohexopyranosyl (1→4)/-O-/3-méthylamino 3,4,6-tridésoxy $\alpha$,D-xylohexopyranosyl (1→6)/N$^1$ (4-amino 2(S)-hydroxy 1-oxo)butyl 2-désoxy D-streptamine et son sulfate.

3. L'O-/2,6-diamino 2,4,6-tridésoxy $\alpha$,D-xylohexopyranosyl (1→4)/-O-/3-méthylamino 3,4,6-tridésoxy $\alpha$,D-xylohexopyranosyl (1→6)/N$^1$ (4-amino 2(S)-hydroxy 1-oxo)butyl 2-désoxy D-streptamine et son sulfate.

4. L' O-/2,6-diamino 2,3,4,6-tétradésoxy $\alpha$,D-érythrohexopyranosyl (1→4)/-O-/3-méthylamino 3,4,6-tridésoxy $\alpha$,D-xylohexopyranosyl (1→6)/N$^1$ (4-amino 2(S)-hydroxy 1-oxo) butyl 2-désoxy D-streptamine et son sulfate.

5. Procédé de préparation des produits de formule I tels que définis à la revendication 1 ainsi que de leurs sels d'addition avec les acides minéraux et organiques, caractérisé en ce que l'on fait réagir un produit de formule II:

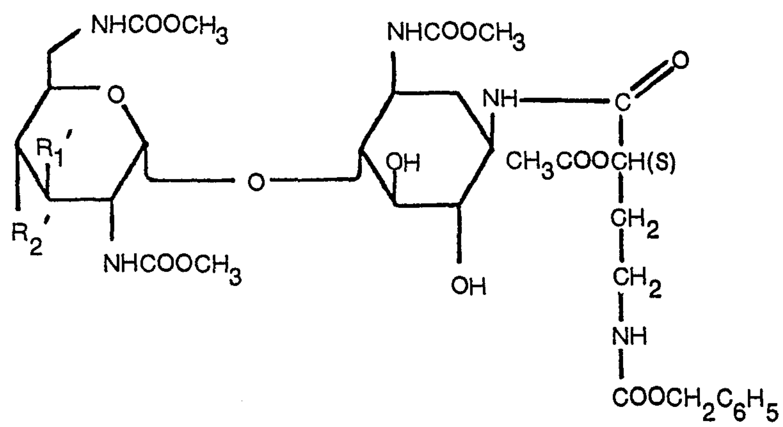

II

dans laquelle les substituants $R'_1$ et $R'_2$ représentent un atome d'hydrogène ou un groupement benzoxy, l'un au moins de ces substituants n'étant pas un groupement benzoxy, avec le produit de formule III:

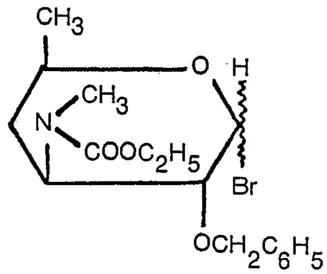

III

en présence d'un agent alcalin, pour obtenir un mélange d'anomères $\alpha$ et $\beta$ de formules IV et IV':

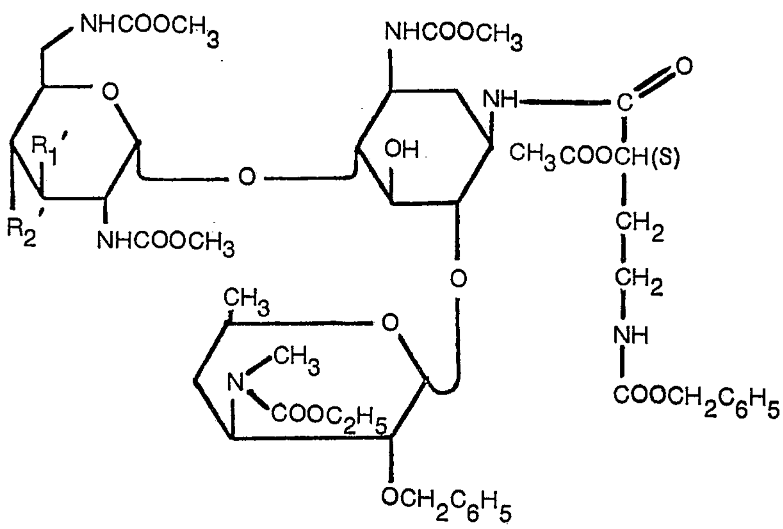

IV

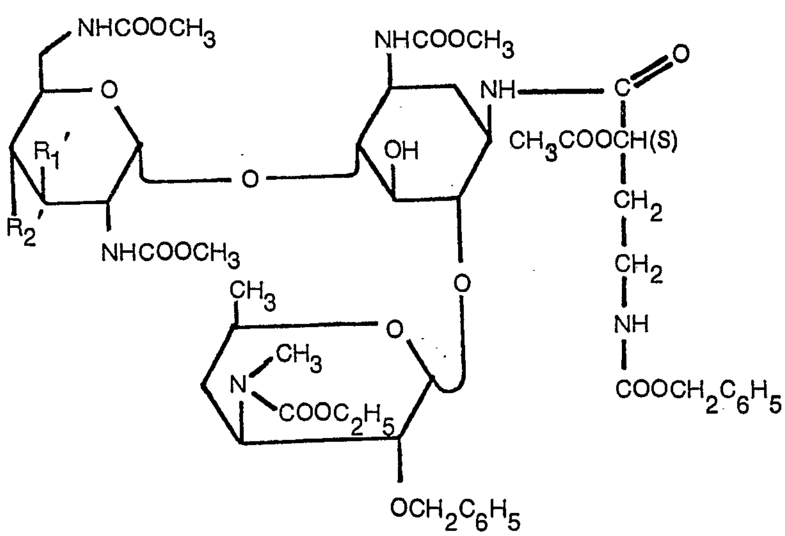

IV'

mélange dont on isole le produit de formule IV, produit de formule IV que l'on hydrolyse en milieu alcalin, dans un solvant organique, pour obtenir un produit de formule V:

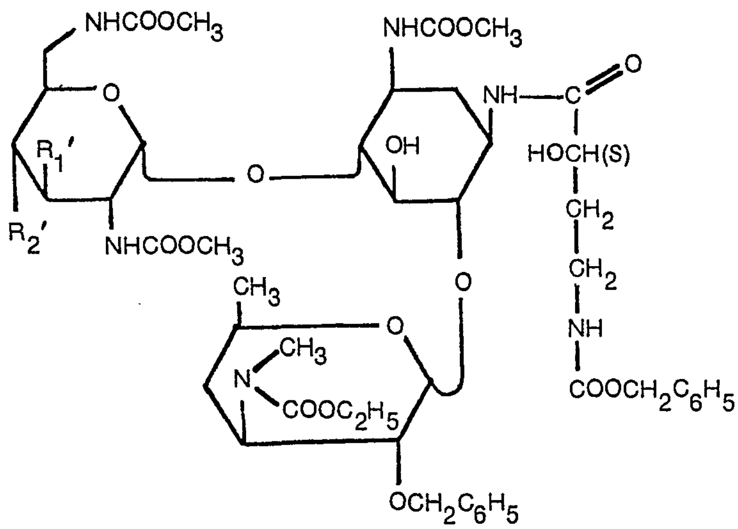

V

produit de formule V que l'on traite par l'hydrogène en présence d'un catalyseur pour obtenir un produit de formule VI:

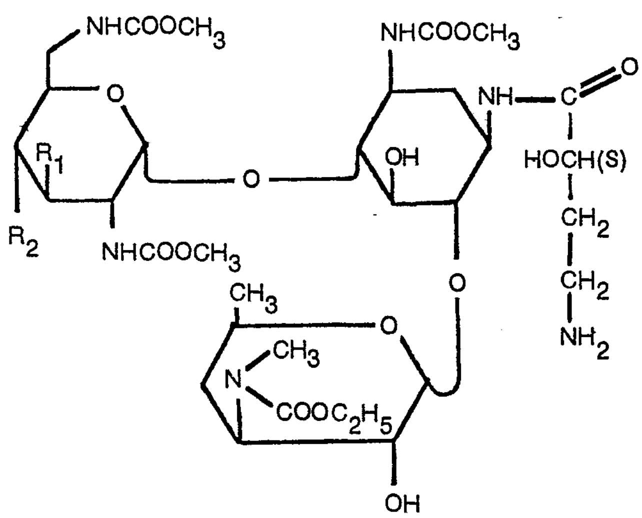

VI

dans aquelle les substituants $R_1$ et $R_2$ ont la signification déjà indiquée, produit, de formule VI que l'on hydrolyse en milieu alcalin pour obtenir le produit de formule I cherché, produit de formule I que l'on salifie, si désiré, par action d'un acide minérale ou organique pour en former le sel cherché.

6. Médicaments et notamment médicaments antibiotiques, caractérisés en ce qu'ils sont constitués par les produits de formule I de la revendication 1 ainsi que par leurs sels thérapeutiquement compatibles.

7. Médicaments et notamment médicaments antibiotiques, caractérisés en ce qu'ils sont constitués par un des produits des revendications 2, 3 ou 4.

8. Compositions pharmaceutiques, caractérisées en ce qu'elles contiennent, à titre de principe actif, l'un au moins des médicaments tels que définis à l'une des revendications 6 ou 7.

**Patentansprüche**

1. Neue, sich von Desoxystreptamin ableitende Aminoglycoside sowie deren Additionssalze mit Mineral- oder organischen Säuren, dadurch gekennzeichnet, daß sie der allgemeinen Formel I

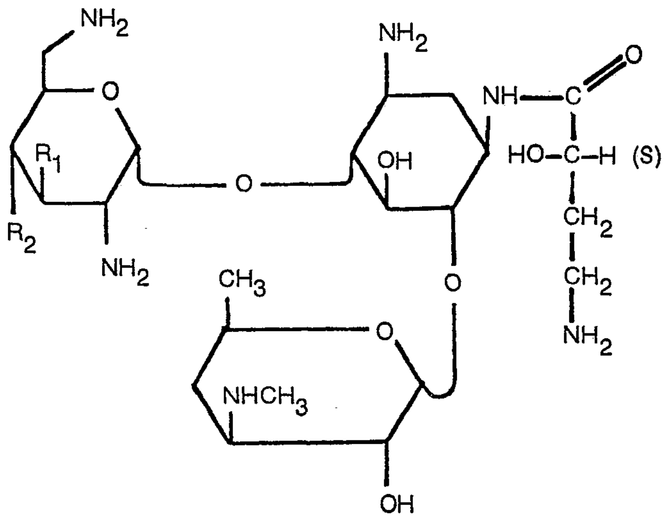

I

entsprechen, worin die Substituenten $R_1$ und $R_2$ ein Wasserstoffatom oder eine Hydroxylgruppe bedeuten, wobei zumindest einer dieser Substituenten keine Hydroxylgruppe ist.

2. O - [2,6 - Diamino - 2,3,6 - tridesoxy - $\alpha$,D - ribohexopyranosyl(1→4)] - O - [3 - methylamino - 3,4,6 - tridesoxy - $\alpha$,D - xylohexopyranosyl(1→6)] - N[1] - (4 - amino - 2(S) - hydroxy - 1 - oxo) - butyl - 2 - desoxy - D - streptamin und sein Sulfat.

3. O - [2,6 - Diamino - 2,4,6 - tridesoxy - $\alpha$,D - xylohexopyranosyl(1→4)] - O - [3 - methylamino-3,4,6 - tridesoxy - $\alpha$,D - xylohexopyranosyl(1→6)] - N[1] - (4 - amino - 2(S) - hydroxy - 1 oxo) - butyl - 2-desoxy - D- streptamin und sein Sulfat.

4. O - [2,6 - Diamino - 2,3,4,6 - tetradesoxy - $\alpha$,D - erythrohexopyranosyl(1→4)] - O - [3 - methylamino - 3,4,6 - tridesoxy - $\alpha$,D - xylohexopyranosyl(1→6)] - N[1] - (4 - amino - 2(S) - hydroxyl - 1- oxo)-butyl - 2 - desoxy - D- streptamin und sein Sulfat.

5. Verfahren zur Herstellung der Produkte der Formel I gemäß Anspruch 1, sowie von deren Additionssalzen mit Mineral- und organischen Säuren, dadurch gekennzeichnet, daß man ein Produkt der Formel II

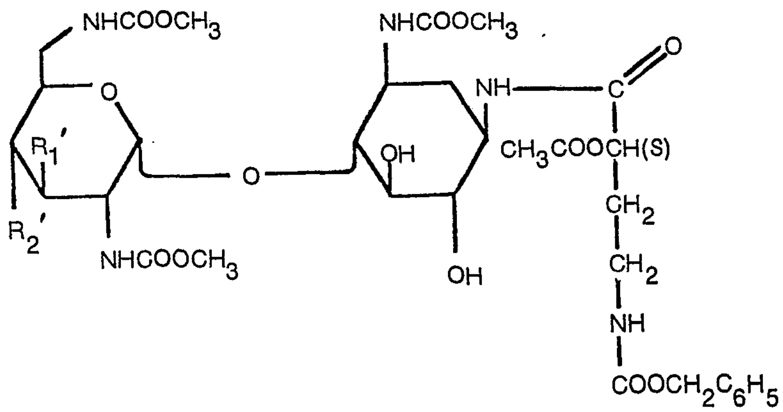

II

worin die Substituenten $R'_1$ und $R'_2$ ein Wasserstoffatom oder eine Benzoxygruppe bedeuten, wobei zumindest einer dieser Substituenten keine Benzoxygruppe ist, mit dem Produkt der Formel III

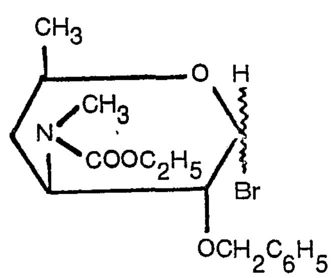

III

in Gegenwart eines alkalischen Mittels umsetzt, um ein Gemisch der $\alpha$- und $\beta$-Anomeren der Formeln IV and IV'

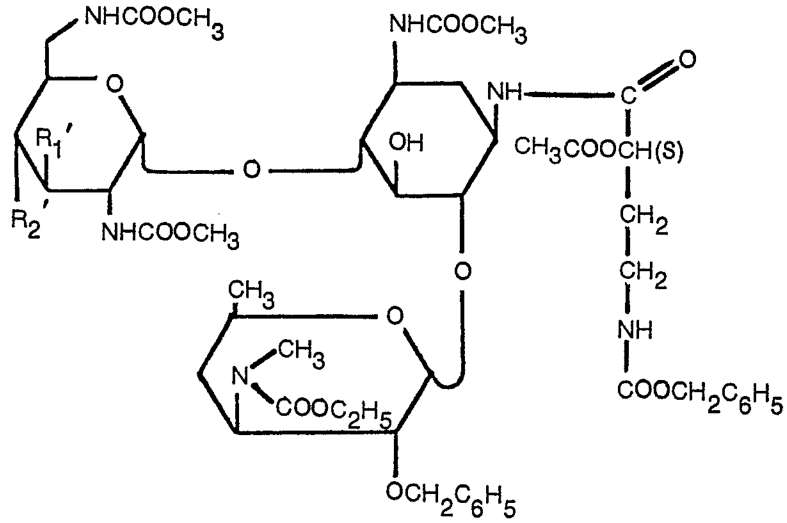

IV

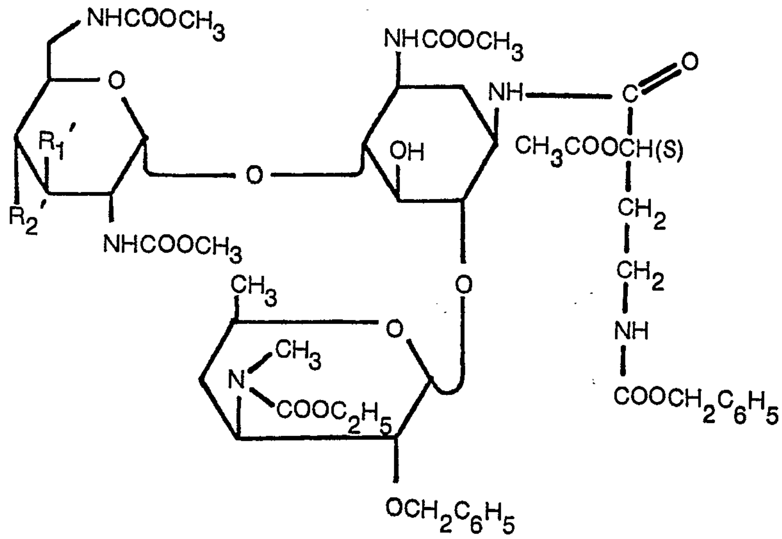

IV'

zu erhalten; aus dem Gemisch das Produkt der Formel IV isoliert, das man in alkalischem Milieu in einem organischen Lösungsmittel hydrolysiert, um eine Produkt der Formel V

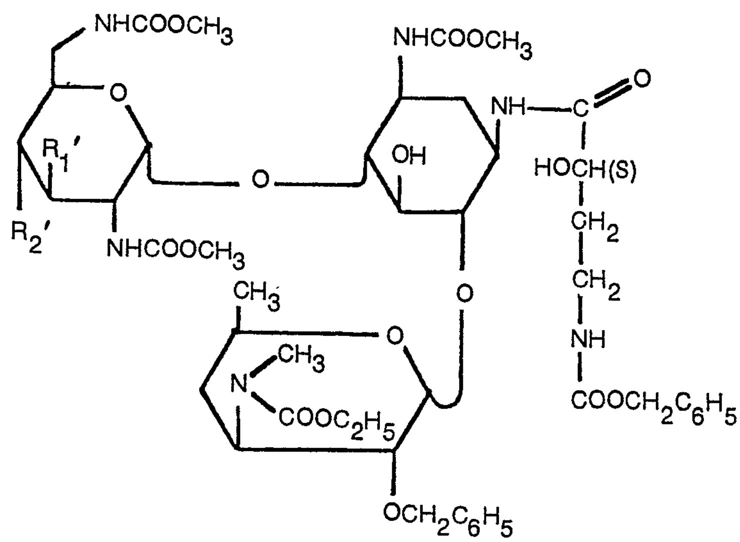

V

zu erhalten, das man mit Wasserstoff in Gegenwart eines Katalysators behandelt, um ein Produkt der Formel VI

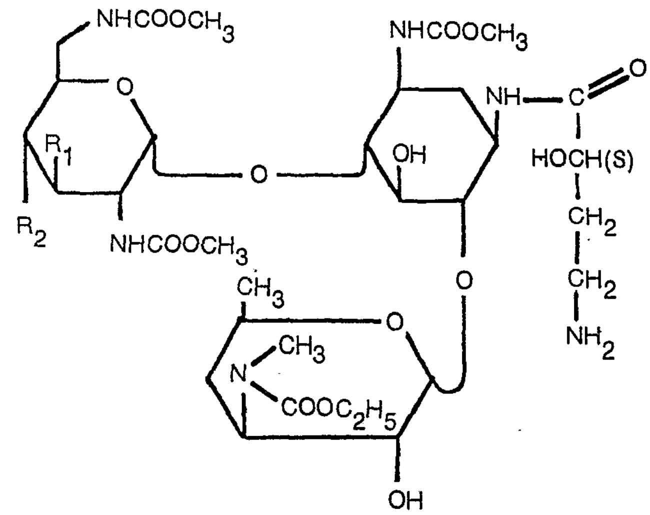

VI

in dem die Substituenten $R_1$ und $R_2$ die angegebene Bedeutung besitzen, zu erhalten, dieses Produkt der Formel VI in alkalischem Milieu hydrolysiert, um das gewünschte Produkt der Formel I zu erhalten, das man gewünschtenfalls durch Umsetzung mit einer Mineral- oder organischen Säure in ein Salz überführt, um das gewünschte Salz zu bilden.

6. Arzneimittel und insbesondere antibiotische Arzneimittel, dadurch gekennzeichnet, daß sie aus den Produkten der Formel I gemäß Anspruch 1, sowie deren therapeutisch verträglichen Salzen bestehen.

7. Arzneimittel und insbesondere antibiotische Arzneimittel, dadurch gekennzeichnet, daß sie aus einem der Produkte der Ansprüche 2, 3 oder 4 bestehen.

8. Pharmazeutische Zusammensetzungen, dadurch gekennzeichnet, daß sie als Wirkstoff zumindest eines der Arzneimittel gemäß einem der Ansprüche 6 oder 7 enthalten.

**Claims**

1. New aminoglycosides derived from desoxy streptamine, as well as their addition salts with mineral or organic acids, characterised in that they correspond to the general formula (I):

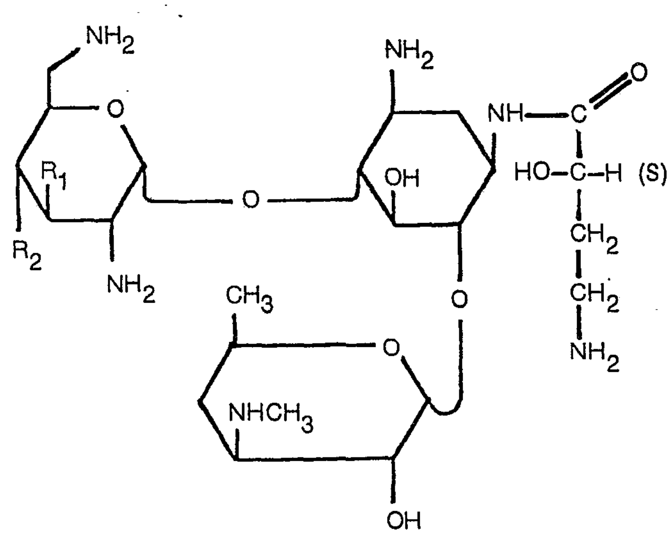

I

in which the substituents $R_1$ and $R_2$ represent a hydrogen atom or a hydroxyl group, one at least of these substituents not being a hydroxyl group.

2. O-/2,6-diamino 2,3,6-tridesoxy $\alpha$,D-ribohexopyranosyl (1→4)/-O-/3-methylamino 3,4,6-tridesoxy $\alpha$,D-xylohexopyranosyl (1→6)/N[1] (4-amino 2(S)-hydroxy 1-oxo)butyl 2-desoxy D-streptamine and its sulphate.

3. O-/2,6-diamino 2,4,6-tridesoxy α,D-xylohexopyranosyl (1→4)/O-/3-methylamino 3,4,6 - tridesoxy α,D-xylohexopyranosyl (1→6)/N[1] (4-amino 2(S)-hydroxy 1-oxo)butyl 2-desoxy D-streptamine and its sulphate.

4. O-/2,6-diamino 2,3,4,6-tetradesoxy α,D-erythrohexopyranosyl (1→4)/-O-/3-methylamino 3,4,6-tridesoxy α,D-xylohexopyranosyl (1→6)/N[1] (4-amino 2(S)-hydroxy 1-oxo)butyl 2-desoxy D-streptamine and its sulphate.

5. Process for preparing the products of formula I as defined in Claim 1, as well as their addition salts with mineral and organic acids, characterised in that a product of formula II:

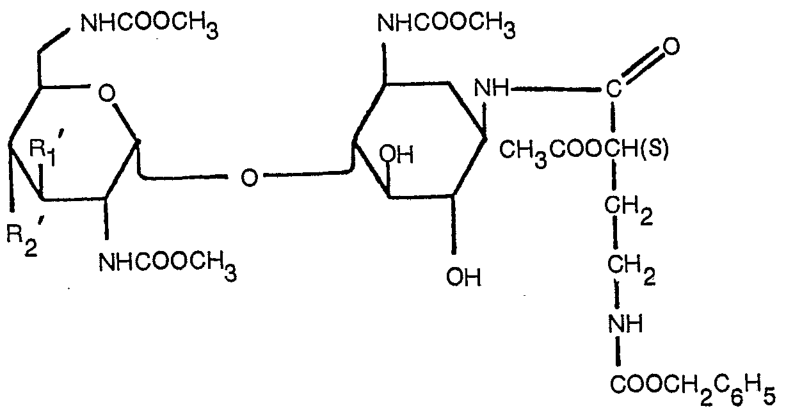

II

in which the substituents $R'_1$ and $R'_2$ represent a hydrogen atom or a benzoxy group, one at least of these substituents not being a benzoxy group, is reacted with the product of formula III:

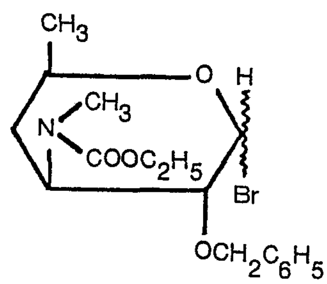

III

in the presence of an alkaline agent, to obtain a mixture of α and β anomers of formulae IV and IV':

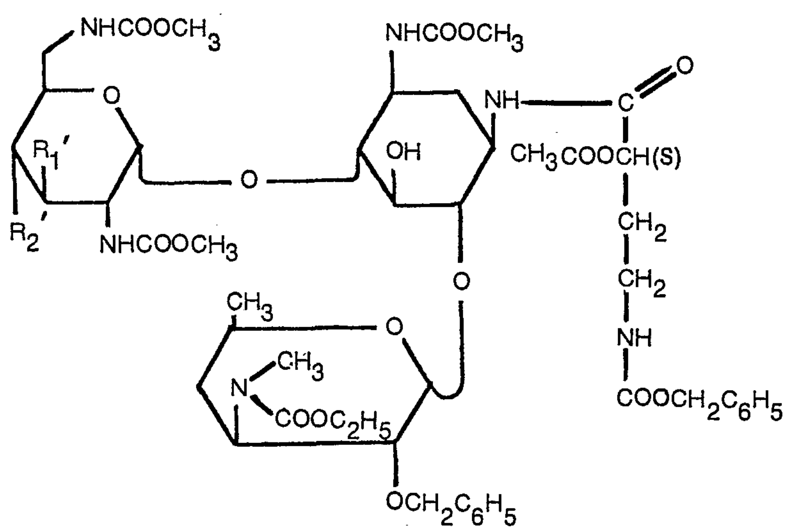

IV

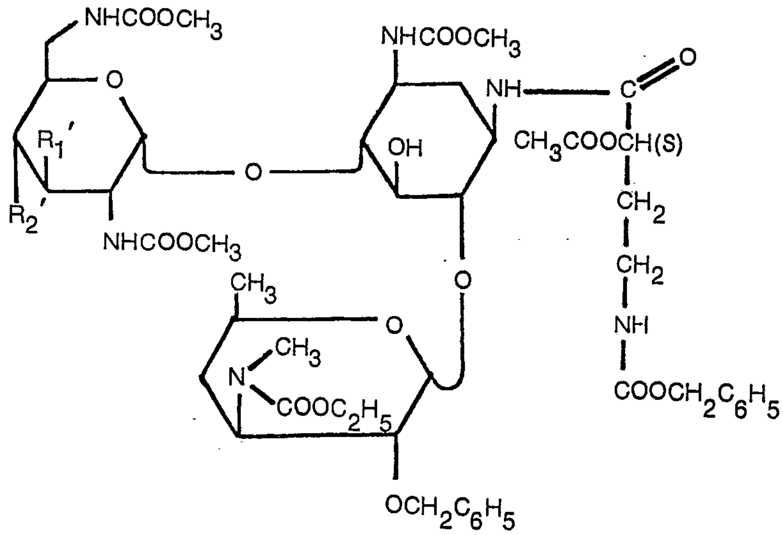

IV′

from which mixture the product of formula IV is isolated and hydrolysed in alkaline medium in an organic solvent to obtain a product of formula V:

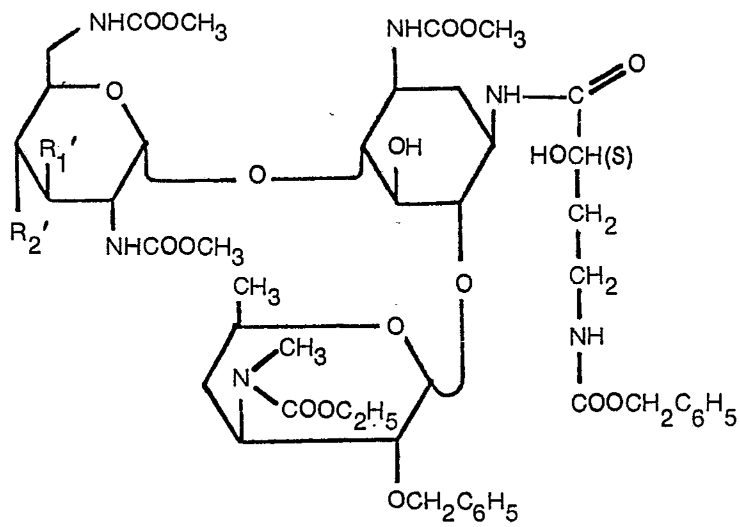

V

which product of formula V is treated with hydrogen in the presence of a catalyst to obtain a product of formula VI:

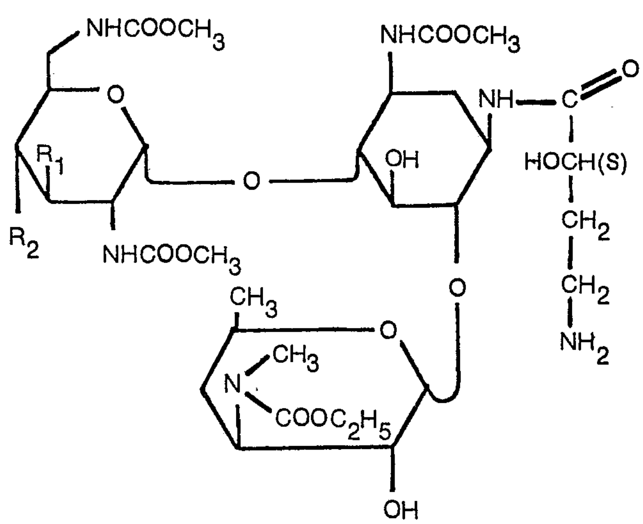

VI

in which the substituents $R_1$ and $R_2$ have the meaning already indicated, which product of formula VI is hydrolysed in alkaline medium to obtain the product sought of formula I, which product of formula I is salified, if desired, by the action of a mineral or organic acid to form the salt sought thereof.

6. Medicaments and especially antibiotic medicaments, characterised in that they are constituted by the products of formula I of Claim 1 as well as by their therapeutically-compatible salts.

7. Medicaments and especially antibiotic medicaments, characterised in that they are constituted by one of the products of Claims 2, 3 or 4.

8. Pharmaceutical compositions, characterised in that they contain, as active principle, one at least of the medicaments as defined in one of Claims 6 or 7.